(19) Europäisches Patentamt
European Patent Office
Office européen des brevets

(11) **EP 3 166 580 B1**

(12) **FASCICULE DE BREVET EUROPEEN**

(45) Date de publication et mention
de la délivrance du brevet:
**18.03.2020 Bulletin 2020/12**

(51) Int Cl.:
*A61K 8/34* *(2006.01)*    *A61K 8/60* *(2006.01)*
*A61Q 13/00* *(2006.01)*    *A61Q 15/00* *(2006.01)*
*A61K 8/73* *(2006.01)*    *A61K 8/11* *(2006.01)*
*A61K 8/92* *(2006.01)*

(21) Numéro de dépôt: **15732729.7**

(22) Date de dépôt: **01.07.2015**

(86) Numéro de dépôt international:
**PCT/EP2015/065010**

(87) Numéro de publication internationale:
**WO 2016/005250 (14.01.2016 Gazette 2016/02)**

(54) **COMPOSITION SOLIDE ANHYDRE À BASE DE PARTICULES ENCAPSULANT UN AGENT BÉNÉFIQUE**

FESTE WASSERFREIE KOSMETISCHE ODER DERMATOLOGISCHE ZUBEREITUNG BEZOGEN AUF FREISETZUNGSPARTIKELN ENHALTEND PFLEGEMITTEL

SOLID ANHYDROUS COSMETIC OR DERMATOLOGIC COMPOSITION BASED ON DELIVERY PARTICLES CONTAINING A BENEFIT AGENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.07.2014 FR 1456634**

(43) Date de publication de la demande:
**17.05.2017 Bulletin 2017/20**

(73) Titulaire: **L'Oréal**
**75008 Paris (FR)**

(72) Inventeurs:
• **MALLE, Gérard**
  **F-77580 Villiers / Smorin (FR)**
• **LUUKAS, Tiina**
  **F-93270 Sevran (FR)**
• **LAVERRE, Didier**
  **F-94152 Chevilly La Rue (FR)**
• **BARA, Isabelle**
  **F-94210 La Varenne St Hilaire (FR)**

(74) Mandataire: **L'Oreal**
**Service D.I.P.I.**
**9, rue Pierre Dreyfus**
**92110 Clichy (FR)**

(56) Documents cités:
**DE-A1-102008 035 013    JP-A- 2008 156 236**
**US-A- 3 971 852    US-A- 5 508 259**
**US-A1- 2004 175 404**

• **MARTIN ET AL.: "Encapsulation and Co-Precipitation Processes with Supercritical Fluids:Applications with Essential Oils", THE OPEN CHEMICAL ENGINEERING JOURNAL, vol. 4, 2010, pages 31-41, XP002737531,**

Remarques:
Le dossier contient des informations techniques présentées postérieurement au dépôt de la demande et ne figurant pas dans le présent fascicule.

**Description**

La présente invention concerne une composition anhydre solide comprenant:

1) au moins des particules comprenant un cœur contenant au moins un agent bénéfique et une enveloppe entourant le coeur; ladite enveloppe comprenant au moins un polysaccharide modifié hydrophobe choisi parmi les $C_5$-$C_{20}$-alcényl succinate d'amidon et au moins un glucide hydrosoluble choisi parmi les maltodextrines de Dextrose Equivalent allant de 4 à 20;
lesdites particules présentant simultanément une densité de poudre versée allant de 300,0g/l à 600,0g/l et une densité absolue supérieure à 1,0;
les particules étant sphériques, et ayant un diamètre moyen en nombre allant de 1 à 30$\mu$m, et un diamètre moyen en volume allant de 5 à 150$\mu$m, et
2) au moins un agent structurant.

L'invention concerne également un procédé cosmétique de soin et/ou d'hygiène et/ou de de conditionnement et/ou de parfumage et/ou de maquillage d'une matière kératinique consistant à appliquer sur ladite matière kératinique une composition telle que définie précédemment.

La présente invention concerne également un procédé cosmétique de traitement des odeurs corporelles et éventuellement de la transpiration humaine consistant à appliquer sur une matière kératinique une composition telle que définie précédemment comprenant au moins un actif déodorant et/ou un actif anti-transpirant sous forme libre et/ou sous forme encapsulée.

De nombreuses formes galéniques cosmétiques permettent de délivrer des agents bénéfiques notamment dans des produits cosmétiques incluant aussi bien des formulations pour le soin et/ou l'hygiène et/ou le maquillage de la peau, des ongles, cils, sourcils ou des cheveux; des produits pour la parfumerie; des produits pour l'hygiène buccale tels que des produits pour le soin de la bouche, des désodorisants tels que chewing-gums, bonbons, pastilles pour l'haleine; des produits pharmaceutiques; des produits à usage vétérinaire comme les litières pour animaux; des produits d'hygiène et/ou le soin des animaux; des produits d'entretien ménager comme les produits pour le soin et/ou le nettoyage du linge (lessives, adoucissants), les produits de vaisselle, les produits de nettoyage et/ou d'entretien des appareils électroménagers, les produits de nettoyage et/ou d'entretien des sols, carrelages, bois etc; des produits sanitaires comme des désodorisants, des produits détartrants, des produits déboucheurs de canalisations ; les produits d'entretien des matières textiles ; des produits d'entretien de la maroquinerie comme les chaussures, les semelles; des produits issus de l'industrie alimentaire; des produits issus de l'agriculture; des produits phyto-sanitaires; des produits issus de l'industrie du bois et du papier.

Parmi celles-ci, les compositions solides constituent une catégorie de produits appréciée par les consommateurs pour leur facilité d'utilisation, de stockage et de conservation. Elles sont largement utilisées dans l'industrie alimentaire (confiserie, biscuiterie), dans les produits d'entretien des matières textiles (cirages, imperméabilisants) et ou encore dans les produits d'entretien et sanitaires (savons, blocs désodorisants, blocs détartrants)

En cosmétique, elles sont en particulier utilisées dans le domaine des déodorants et anti-transpirants sous forme de bâtonnets ou sticks mais peuvent également être valorisées dans des produits de maquillage ou de soin des matières kératiniques humaines comme la peau, ou les lèvres tels que rouges à lèvres, les fonds de teint coulés en stick ou en coupelle, les fards à joues ou paupières, les stick anti-cernes, brillants à lèvres, eye-liners, mascaras, baumes ou bases de soin pour les lèvres, onguents pour le corps, baumes ou bases de soins journaliers ou encore sticks solaires ou auto-bronzants.

Le but de la présente invention est de proposer de nouvelles compositions de type solide anhydre comprenant au moins un agent bénéfique encapsulé dans des particules qui seraient étanches à l'abri de l'humidité c'est-à-dire inodores si l'actif est un parfum

- les dites particules présentant une densité de poudre versée faible pour faciliter leur formulation et conserver une texture légère et douce
- lesdites particules devant également être compatibles avec les ingrédients habituels de ces formulations et suffisamment résistantes pour pouvoir être formulées en solide sans être endommagées
- ledit agent bénéfique contenu dans les particules pouvant être libéré de façon quasi immédiate, progressive et répétable sur la peau, le cheveu et les phanères au contact de l'eau

On sait qu'il existe une nécessité dans de nombreux domaines industriels, de protéger un certain nombre de molécules fragiles ou volatiles et de contrôler leur relargage vers un milieu extérieur.

Un des moyens permettant d'atteindre un tel but est leur encapsulation. Cette encapsulation a pour objectif de diminuer l'évaporation et le transfert vers l'environnement de la matière active, soit au cours du stockage, soit au cours de l'élaboration des produits ou encore au cours de leur utilisation. Elle peut également permettre de rendre le matériau plus facile d'utilisation en le diluant et en favorisant sa répartition homogène au sein du support.

La micro-encapsulation regroupe l'ensemble des technologies permettant l'enrobage ou le piégeage de principes actifs sous forme solide, liquide, ou gazeuse au sein de particules individualisées dont la taille s'échelonne entre quelques microns et quelques millimètres. Si ces microparticules sont creuses (vésiculaires) on parle de microcapsules, si elles sont pleines (matricielles) on parle de microsphères. Leur taille varie de $1\mu m$ à plus de $1000\mu m$. Ces microparticules peuvent être biodégradables ou non et peuvent contenir entre 5 et 90% (en masse) de substance active.

Les substances actives encapsulées sont d'origines très variées: principes actifs pharmaceutiques, cosmétiques, additifs alimentaires, produits phytosanitaires, essences parfumées, micro-organismes, cellules, ou encore catalyseurs de réaction chimique...

Tout l'intérêt des microparticules d'encapsulation réside dans la présence d'une membrane polymérique, qui isole et protège le contenu du milieu extérieur. Selon les cas, la membrane sera détruite lors de l'utilisation pour libérer son contenu (exemple : encarts publicitaires "scratch and sniff" libérant le parfum lorsqu'on écrase les microcapsules), ou bien la membrane restera présente tout le long de la libération du contenu, dont elle contrôlera la vitesse de diffusion (exemple: encapsulation de médicaments pour libération ralentie).

Les matériaux d'enrobage sont généralement des polymères d'origine naturelle ou synthétique, hydrophobes ou hydrophiles, ou bien des lipides.

Les principaux procédés pour réaliser l'encapsulation de substances dans des microparticules sont la polymérisation interfaciale, la réticulation interfaciale, l'émulsion suivie d'une évaporation ou d'une extraction du solvant, la double émulsion évaporation/extraction de solvant, le spray-drying, le prilling, la coacervation (JP 2008 156236 A).

Dans le brevet US 5 508 259, on a proposé des compositions parfumantes non aqueuses, comprenant des parfums encapsulés dans des particules sphériques (capsules) solubles dans l'eau. Lesdites particules sont obtenues par des techniques d'encapsulation conventionnelles et en particulier le spray-drying d'une émulsion constituée d'un substrat solide filmogène en combinaison avec un agent émulsionnant et un mélange d'ingrédients de parfumerie. Le substrat solide filmogène est notamment choisi parmi l'acétate de polyvinyle, l'alcool polyvinylique, des dextrines, de l'amidon naturel ou modifié, les gommes végétales, les pectines, les xanthanes, les alginates, carraghénanes ou encore les dérivés de cellulose tels que par exemple la carboxyméthylcellulose, la méthylcellulose ou l'hydroxyéthylcellulose. L'émulsion est ensuite déshydratée par un procédé classique d'atomisation (spray-drying), qui consiste, tel que décrit à l'exemple 1, à la pulvériser en fines gouttelettes dans un atomiseur avec un débit de 50kg/h et une pression de 0,45 bars, au contact d'un courant d'air de $320 m^3/h$ chauffé à 350°C afin d'évaporer l'eau, ce qui permet d'obtenir une poudre fine avec un diamètre de particules compris entre 20 et 80 microns et contenant 20% en poids de parfum.

Cependant, on a remarqué que les particules obtenues par ce procédé étaient fortement odorantes à l'état sec à cause de la présence de parfum libre (non encapsulé), qu'elles étaient principalement constituées d'agglomérats pouvant nuire à l'homogénéité du produit et et gêner la bonne application du produit et ne possédaient pas les caractéristiques de densité adaptées à l'objectif de l'invention.

Dans le brevet US 6 200 949, on a décrit également un procédé de formation d'une matière particulaire contenant un parfum hydrophile comprenant les étapes successives consistant à former une émulsion aqueuse de parfum contenant 40 à 60% en poids d'eau, 3 à 30% en poids de maltodextrine, 10 à 40% en poids d'amidon modifié hydrophobe, puis à la sécher par pulvérisation dans un atomiseur (courant d'air de $420 m^3/h$ chauffé à 204°C de sorte que les particules sont formées avec une taille moyenne d'environ 3 à environ 10 microns et une teneur en parfum de 15 à 50% en poids. Cependant, les particules obtenues par ce procédé sont fortement odorantes à l'état sec à cause de la présence de parfum libre (non encapsulé), sont principalement constituées d'agglomérats, peuvent nuire à l'homogénéité du produit et ne possèdent pas les caractéristiques de densité adaptées à l'objectif de l'invention.

Il est donc très important de pouvoir disposer de particules étanches qui ne libèrent leur contenu qu'à la demande (en réponse à l'humidité ambiante, notamment dans les zones climatiques humides, en réponse à la transpiration corporelle, au shampooing ou sous la douche, etc...), d'une part pour garantir la protection dans le temps de l'actif encapsulé surtout s'il est fragile et/ou volatil et d'autre part pour éviter les interactions avec les autres ingrédients de la formule. Lorsque l'agent bénéfique encapsulé est un ingrédient de parfumerie et/ou un parfum complet, il est d'autant plus important que l'encapsulation soit totale, ce qui conduit à des particules inodores en formules anhydres permettant au formulateur de les associer ou non avec n'importe quel un parfum libre de son choix (identique ou différent) sans risque d'interactions ou de perturbation de la note parfumée choisie.

Dans le brevet EP1917098B1, on a proposé un procédé de préparation de produits d'encapsulation par précipitation, lequel procédé emploie:

- une émulsion pouvant être pompée comprenant (i) une phase continue contenant un solvant et un soluté formant une matrice dissout dans ledit solvant et (ii) une phase dispersée;

- un extracteur comprenant un gaz supercritique, sous-critique ou liquéfié;

ledit solvant étant sensiblement plus soluble dans l'extracteur que ledit soluté formant une matrice et ledit procédé comprenant les étapes successives consistant à:

a. combiner l'émulsion pouvant être pompée avec l'extracteur dans des conditions de mélange;

b. permettre la formation de produits d'encapsulation particulaires dans lesquels la phase dispersée est imbriquée dans une matrice solide du soluté formant une matrice;

c. collecter les produits d'encapsulation et les séparer de l'extracteur.

Il est indiqué que ce procédé peut être utilisé dans les industries pharmaceutique et agroalimentaire ainsi-que dans les domaines de l'agriculture, du «coating», des adhésifs et des catalyseurs. Il peut en particulier être utilisé pour encapsuler des actifs pharmaceutiques, des arômes, des enzymes, des colorants, des pesticides et des herbicides.

Après d'importantes recherches, la demanderesse a découvert de façon surprenante et inattendue qu'il était possible d'atteindre les objectifs tels qu'énoncés précédemment en utilisant, dans une composition anhydre solide comprenant au moins un agent structurant et des particules comprenant un cœur contenant au moins un agent bénéfique et une enveloppe entourant le cœur; ladite enveloppe comprenant au moins un polysaccharide modifié hydrophobe choisi parmi les $C_5$-$C_{20}$-alcényl succinate d'amidon et au moins un glucide hydrosoluble choisi parmi les maltodextrines de D.E. allant de 4 à 20; lesdites particules présentant simultanément une densité de poudre versée allant de 300,0g/l à 600,0g/l et une densité absolue supérieure à 1,0 ; et les particules étant sphériques, et ayant un diamètre moyen en nombre allant de 1 à 30$\mu$m, et un diamètre moyen en volume allant de 5 à 150um. Ces particules peuvent être en particulier obtenues par le procédé tel que décrit dans le brevet EP1917098B1 commenté précédemment.

Les particules conformes à la présente invention permettent d'encapsuler des ingrédients bénéfiques, en particulier fragiles, de façon complète (encapsulation totale) sans dégradation dans des capsules qui seraient suffisamment résistantes et étanches pour pouvoir être conservées sans altération à l'abri de l'humidité et pouvant être aisément formulées et rester stables dans des compositions anhydres solides. Ces mêmes particules dans ce type de composition présentent de préférence une morphologie sphérique et une densité de poudre versée très faible pour conserver la texture légère et douce; elles ont également la capacité de s'ouvrir en présence d'eau pour pouvoir libérer leur agent bénéfique de façon quasi immédiate, progressive et répétable sur la peau, le cheveu et les phanères au contact de l'eau

Cette découverte est à la base de la présente invention.

La présente invention concerne une composition anhydre solide comprenant:

1) au moins des particules comprenant un cœur contenant au moins un agent bénéfique et une enveloppe entourant le coeur; ladite enveloppe comprenant au moins un polysaccharide modifié hydrophobe choisi parmi les $C_5$-$C_{20}$-alcényl succinate d'amidon et au moins un glucide hydrosoluble choisi parmi les maltodextrines de DE allant de 4 à 20; lesdites particules présentant simultanément une densité de poudre versée allant de 300,0g/l à 600,0g/l et une densité absolue supérieure à 1.0, les particules étant sphériques, et ayant un diamètre moyen en nombre allant de 1 à 30$\mu$m, et un diamètre moyen en volume allant de 5 à 150$\mu$m; et
2) au moins un agent structurant.

De préférence, la composition comprend un milieu physiologiquement acceptable.

Selon une forme particulière de l'invention, les compositions sont cosmétiques ou dermatologiques.

Selon une forme particulière de l'invention, les compositions selon l'invention peuvent être utilisées dans d'autres applications industrielles et notamment être des produits de consommation choisis parmi des produits pour l'hygiène buccale tels que des produits pour le soin de la bouche, des désodorisants tels que chewing-gums, bonbons, pastilles pour l'haleine ; des produits ; des produits à usage vétérinaire comme les litières pour animaux ; des produits d'hygiène et/ou le soin des animaux ; des produits d'entretien ménager comme les produits pour le soin et/ou le nettoyage du linge (lessives, adoucissants), les produits de vaisselle, les produits de nettoyage et/ou d'entretien des appareils électroménagers, les produits de nettoyage et/ou d'entretien des sols, carrelages, bois etc ; des produits sanitaires comme des désodorisants, des produits détartrants , des produits déboucheurs de canalisations ; les produits d'entretien des matières textiles; des produits d'entretien de la maroquinerie comme les chaussures, les semelles; des produits issus de l'industrie agro-alimentaire; des produits issus de l'agriculture; des produits phyto-sanitaires; des produits issus de l'industrie du bois et du papier.

L'invention concerne également un procédé cosmétique de soin et/ou d'hygiène et/ou de de conditionnement et/ou de parfumage et/ou de maquillage d'une matière kératinique consistant à appliquer sur ladite matière kératinique une composition telle que définie précédemment.

L'invention concerne encore un procédé de traitement cosmétique des odeurs corporelles et éventuellement de la transpiration humaine consistant à appliquer sur une matière kératinique une composition telle que définie précédemment comprenant au moins un actif déodorant et/ou un actif anti-transpirant sous forme libre et/ou sous forme encapsulée.

L'invention concerne aussi un produit de consommation, caractérisé par le fait qu'il est constitué par une composition telle que définie précédemment.

**Définitions**

Par «anhydre», on entend au sens de la présente invention, une phase liquide présentant une teneur en eau inférieure à 5% en poids, de préférence inférieure à 2% en poids et de manière encore plus préférée inférieure à 1% en poids par rapport au poids de ladite composition voire encore moins de 0,5% et notamment exempte d'eau, l'eau n'étant pas ajoutée lors de la préparation de la composition mais correspondant à l'eau résiduelle apportée par les ingrédients mélangés

Au sens de la présente invention, on entend désigner par «milieu physiologiquement acceptable», un milieu convenant à l'administration d'une composition par voie topique. Un milieu physiologiquement acceptable est sans odeur et/ou sans aspect désagréable, et qui est parfaitement compatible avec la voie d'administration topique.

Par "matière kératinique", on entend la peau, les cuirs, le cuir chevelu, les lèvres, et/ou les phanères tels que les ongles et les fibres kératiniques, telles que par exemple, les poils, les cils, les sourcils et les cheveux.

Par «composition cosmétique», on entend au sens de l'invention toute composition appliquée sur une matière kératinique pour produire un effet non-thérapeutique d'hygiène, de soin, de conditionnement ou de maquillage contribuant à l'amélioration du bien-être et/ou à l'embellissement et/ou la modification de l'aspect de la matière kératinique sur laquelle on applique ladite composition.

Par «composition dermatologique», on entend au sens de l'invention toute composition appliquée sur une matière kératinique pour prévenir et/ou traiter un désordre ou un dysfonctionnement de ladite matière kératinique.

Par «soin et traitement cosmétique», on entend au sens de l'invention tout effet non-thérapeutique d'hygiène, de soin, de conditionnement ou de maquillage contribuant à l'amélioration du bien-être et/ou à l'embellissement et/ou la modification de l'aspect de la matière kératinique sur laquelle on applique ladite composition.

Par «produit de consommation», on entend tout produit fabriqué destiné à être utilisé ou consommé dans la forme où il est commercialisé et qui n'est pas destiné à une fabrication ou modification ultérieure. Sans que les exemples soient limitatifs, les produits de consommation selon l'invention peuvent être des formulations pour le soin et/ou l'hygiène et/ou le maquillage de la peau, des ongles, des cils, des sourcils, des cheveux ou du cuir chevelu; des produits pour la parfumerie; des produits pour l'hygiène buccale tels que des produits pour le soin de la bouche, des désodorisants tels que chewing-gums, bonbons, pastilles pour l'haleine; des produits pharmaceutiques; des produits à usage vétérinaire comme les litières pour animaux; des produits d'hygiène et/ou le soin des animaux; des produits d'entretien ménager comme les produits pour le soin et/ou le nettoyage du linge (lessives, adoucissants), les produits de vaisselle, les produits de nettoyage et/ou d'entretien des appareils électroménagers, les produits de nettoyage et/ou d'entretien des sols, carrelages, bois etc; des produits sanitaires comme des désodorisants, des produits détartrants, des produits déboucheurs de canalisations ; les produits d'entretien des matières textiles; des produits d'entretien de la maroquinerie comme les chaussures, les semelles; des produits issus de l'industrie agro-alimentaire tels que des produits de confiserie (bonbons, chewing-gums) ou de biscuiterie; des produits issus de l'agriculture; des produits phyto-sanitaires; des produits issus de l'industrie du bois et du papier.

Par «agent bénéfique», on entend au sens de l'invention tout composé chimique présent dans un produit de consommation produisant un effet bénéfique perçu par le consommateur lors de son utilisation et/ou obtenu sur le produit de consommation lui-même, ledit effet bénéfique pouvant être une amélioration sensorielle ou une modification notamment visuelle et/ou olfactive, et/ou gustative et/ou tactile, une amélioration du confort et /ou de la facilité d'application, un effet esthétique, un effet hygiénique, une sensation de propreté, un effet curatif et/ou prophylactique.

Par «particules comprenant un cœur contenant au moins un agent bénéfique», on entend une particule comprenant au moins un agent bénéfique immobilisé, capturé et/ou encapsulé dans la matrice d'un système d'encapsulation ou de piégeage; ledit agent bénéfique étant libéré vers l'extérieur au fur et à mesure de la détérioration du système d'encapsulation ou de piégeage lorsque sa dégradation se produit au contact d'un milieu avec lequel il va réagir ou sous l'effet d'un stimulus tel qu'un apport d'eau.

Par «composition solide», on entend que la mesure de la force maximale mesurée en texturométrie lors de l'enfoncement d'une sonde dans l'échantillon de formule doit être au moins égale à 0,25 Newton, en particulier au moins égal à 0,30 Newton, notamment au moins égale 0,35 Newton, appréciée dans des conditions de mesure précises comme suit.

Les formules sont coulées à chaud dans des pots de 4cm de diamètre et 3cm de fond. Le refroidissement est fait à température ambiante. La dureté des formules réalisées est mesurée après 24 heures d'attente. Les pots contenant les échantillons sont caractérisés en texturométrie à l'aide d'un texturomètre tel que celui commercialisé par la société Rhéo TA-XT2, selon le protocole suivant : une sonde de type bille en inox de diamètre 5mm est amenée au contact de l'échantillon à une vitesse de 1mm/s. Le système de mesure détecte l'interface avec l'échantillon avec un seuil de détection égal à 0,005 newtons. La sonde s'enfonce de 0,3mm dans l'échantillon, à une vitesse de 0,1mm/s. L'appareil de mesure enregistre l'évolution de la force mesurée en compression au cours du temps, pendant la phase de pénétration. La dureté de l'échantillon correspond à la moyenne des valeurs maximales de la force détectée pendant la pénétration, sur au moins 3 mesures.

Par «agent structurant», on entend tout composé minéral ou organique, sous forme de molécule simple ou de polymère, capable de rigidifier la composition jusqu'à obtenir une composition solide selon la définition indiquée précédemment.

## Densité de poudre versée (ou densité apparente non tassée)

La détermination est effectuée à température ambiante (20-25°C) et dans les conditions normales atmosphériques (1 atmosphère) à l'aide d'une éprouvette de 100ml. L'éprouvette est pesée vide puis remplie d'un volume de 100ml de poudre versée, sans tassement. La différence de masse entre éprouvette vide et remplie de 100ml de poudre donne la densité de poudre versée.

## Densité absolue

### Principe de la mesure

La mesure consiste à déterminer le poids d'un échantillon du solide en poudre par simple pesée puis à mesurer le volume occupé par les particules de poudre en mesurant le volume de liquide déplacé par l'échantillon de poudre par immersion dans ce liquide. Le liquide choisi doit être peu volatil et ne doit pas être un solvant de la poudre. On choisit généralement le cyclohexane. Les mesures sont réalisées au moins deux fois.

### Matériels :

Une fiole jaugée de 10 ou 25ml et une balance de précision.

- $m_1$ est le poids de la fiole vide
- $m_2$ est le poids de la fiole remplie d'eau jusqu'au trait de jauge.
- $m_3$ est le poids de la fiole remplie de cyclohexane jusqu'au trait de jauge.
- $m_4$ est le poids de la fiole remplie environ au tiers de sa capacité par la poudre à analyser.

On remplit la fiole environ au tiers de sa capacité avec la poudre à analyser.

### Méthode

On complète la fiole, un peu en-dessous du trait de jauge avec du cyclohexane. Afin d'éliminer complètement l'air emprisonné dans la poudre on opère comme suit :

1) on traite la fiole dans un bac à ultra-sons pendant 5 minutes
2) on ajuste le niveau du cyclohexane jusqu'au trait de jauge
3) on traite la fiole dans un bac à ultra-sons pendant 2 minutes
4) les étapes 2 et 3 sont répétées si nécessaire jusqu'à ce que le niveau du cyclohexane n'évolue plus.

$m_5$ est le poids de la fiole ainsi remplie.
Le poids de poudre analysée est égal à $m_4$-$m_1$ (pour une bonne précision ce poids doit être supérieur à 2g). La densité de l'air étant très faible par rapport à celle du solide on admet que $m_4$ - $m_1$ est égal
Le poids de cyclohexane correspondant au volume occupé par le solide (Vs) est égal à :
$m_6 = (m_3 - m_1) - (m_5 - m_4) = \rho_{cyclo}.Vs$ où $\rho_{cyclo}$ est la densité du cyclohexane à la température du laboratoire.
La densité absolue du solide constitutif de la poudre est égale à

$$\rho_{cyclos} = (m_4 - m_1)/Vs = \rho_{cyclo}(m_4 - m_1)/m_6.$$

Si la densité du cyclohexane à la température du laboratoire n'est pas connue, on la détermine comme suit par rapport à celle de l'eau :

Soit Vf le volume jaugé de la fiole et $\rho_{eau}$ la densité de l'eau à la température du laboratoire on a :

$$\rho_{cyclo} = (m_3 - m_1) / Vf \text{ et } \rho_{eau} = (m_2 - m_1) / Vf$$

soit $\rho_{cyclo} = \rho_{eau} (m_2 - m_1) / (m_3 - m_1)$

La densité absolue du solide constitutif de la poudre est égale à :

$$\rho_s = [\rho_{eau} (m_4 - m_1) (m_2 - m_1)] / [m_6 (m_3 - m_1)].$$

## PARTICULES D'ENCAPSULATION

Les particules conformes à l'invention comprennent un cœur contenant au moins un agent bénéfique et une enveloppe entourant le cœur; ladite enveloppe comprenant au moins un polysaccharide modifié hydrophobe choisi parmi les $C_5$-$C_{20}$-alcényl succinate d'amidon et au moins un glucide hydrosoluble choisi parmi les maltodextrines de D.E. allant de 4 à 20; lesdites particules présentant simultanément une densité de poudre versée allant de 300,0 g/l à 600,0 g/l et une densité absolue supérieure à 1,0 ; les particules étant sphériques, et ayant un diamètre moyen en nombre allant de 1 à 30$\mu$m, et un diamètre moyen en volume allant de 5 à 150$\mu$m.

Par « sphériques », on entend que la particule présente un indice de sphéricité, c'est-à-dire le rapport entre son plus grand diamètre et son plus petit diamètre, est inférieur à 1,2. Dans ce cas, de telles particules sont généralement appelées «capsules».

Par « taille moyenne » des particules on entend les paramètres D[4,3] et D[2,3] mesurés en voie sèche par diffraction laser à l'aide d'un granulomètre Microtrac S3500, les résultats étant exprimés sous la forme de distributions granulométriques en volume et en nombre donnant accès au diamètre moyen.

Les particules sphériques conformes à la présente invention présentent ainsi un diamètre moyen en nombre allant de 1 à 30$\mu$m, de préférence allant de 2 à 15$\mu$m et encore mieux de 5 à 10$\mu$m et un diamètre moyen en volume allant de 5 à 150$\mu$m, de préférence allant de 10 à 100$\mu$m et encore mieux de 20 à 80$\mu$m.

Les particules selon l'invention contenant l'agent bénéfique représentent de préférence de 0,1 à 60% en poids, de préférence 0,3 à 40% en poids et encore mieux de 0,5 à 20% en poids du poids total de la composition.

## POLYSACCHARIDE MODIFIE HYDROPHOBE

On entend par «polysaccharide modifié hydrophobe» tout polysaccharide modifié par voie chimique ou enzymatique et comportant au moins un groupe fonctionnel hydrophobe.

Les polysaccharides sont des macromolécules glucidiques formées par l'enchaînement d'un grand nombre de sucres élémentaires (oses) hydrophiles liés entre eux par des liaisons O-osidiques.

Les groupes fonctionnels hydrophobes de la présente invention sont des groupes hydrocarbonés (constitués essentiellement d'atomes de carbone et d'hydrogène) comprenant, de préférence au moins 6 et encore mieux au moins 8 atomes de carbone alcényles. Le nombre maximum d'atomes de carbone du groupe hydrocarboné est de 20, et préférentiellement 18. Les groupes hydrocarbonés hydrophobes peuvent être non substitués, par exemple constitués d'une simple longue chaine alkyle ou bien substitués par des groupes non réactifs comme des groupes aromatiques tels que des groupes aryles (ie: phényle) ou aralkyles(ie: benzyle) ou encore des groupes polaires tels que par exemple des carboxyles ou des hydroxyles.

Pour greffer le ou les groupes fonctionnels hydrophobes sur les polysaccharides, on utilise généralement des dérivés d' acides carboxyliques ou leurs dérivés (esters, halogénures d'acides, anhydrides).

Le polysaccharide modifié hydrophobe représente de préférence de 20 à 90% en poids, notamment 30 à 80% en poids, mieux 40 à 70% en poids, et encore mieux 40 à 60% en poids par rapport au poids total de l'enveloppe de la particule.

Les molécules d'amidons peuvent avoir comme origine botanique les céréales ou encore les tubercules. Ainsi, les amidons sont par exemple choisis parmi les amidons de maïs, de riz, de manioc, de tapioca, d'orge, de pomme de terre, de blé, de sorgho, de pois.

On entend par «amidon modifié hydrophobe» tout amidon modifié par réaction chimique ou enzymatique et comprenant au moins un groupe fonctionnel hydrophobe.

Les amidons modifiés hydrophobes conformes à l'invention sont choisis parmi les $C_5$-$C_{20}$ alcényl succinates d'amidon,_plus particulièrement l'octényle succinate d'amidon sodique (E1450- CAS 66829-29-6 / 52906-93-1 / 70714-61-3), en particulier celui commercialisé par National Starch sous la dénomination CAPSUL®.

On peut également citer les références commerciales CAPSUL TA®, N-LOK®, N- LOK 1930®, HI-CAP 100®, PURITY GUM 1773®, PURITY GUM 2000 ® de National Starch, CLEARGUM CO® de la société Roquette et EMCAP®, EMTEX®, DELITEX de la société Cargill.

## GLUCIDE HYDROSOLUBLE

Par «glucide hydrosoluble», on entend un glucide ou polyol qui, introduit dans l'eau sans modification de pH à 25°C, à une concentration massique égale à 3%, permet l'obtention d'une solution macroscopiquement homogène et transparente, c'est à dire ayant une valeur de transmittance minimum de la lumière, à une longueur d'onde égale à 500nm, à travers un échantillon de 1cm d'épaisseur, d'au moins 80%, de préférence d'au moins 90%.

Par «glucide » (encore appelés hydrates de carbone ou carbohydrates ou saccharides) on entend l'ensemble des sucres simples ou oses et leurs combinaisons ou osides.

Les glucides comprennent habituellement:

(1) les monosaccharides ou oses qui sont de deux types: les aldoses comprenant une fonction aldéhyde sur le premier carbone et les cétoses comprenant une fonction cétone sur le deuxième carbone. On les distingue aussi suivant le nombre d'atomes de carbone qu'ils possèdent.

(2) les oligosaccharides (ou oligosides) qui sont des oligomères d'oses ayant un enchaînement de 2 à 10 unités monosaccharides unies par des liaisons glycosidiques.

(3) Les polyholosides (ou polysaccharides ou polyosides) qui sont des polymères d'oses ayant un enchaînement de monosaccharides supérieur à 10 unités

## GLUCIDES HYDROSOLUBLES

Les glucides hydrosolubles de l'invention sont choisi parmi les maltodextrines les maltodextrines ayant un Dextrose Equivalent allant de 4 à 20.

Les maltodextrines sont le résultat de l'hydrolyse d'un amidon blé, maïs) ou d'une fécule (pomme de terre). Elles sont constituées de différents sucres (glucose, maltose, maltotriose, oligosides et polyosides) directement issus de cette réaction, dans des proportions qui dépendent du degré de l'hydrolyse.

Ce degré est mesuré par «dextrose équivalent», ou D.E, le dextrose étant du D-glucose, c'est le résultat d'une hydrolyse totale de l'amidon. Plus le D.E. est élevé, plus l'hydrolyse est poussée, et donc plus la proportion en sucres simples (à chaîne courte) composant la maltodextrine est élevée.

Les maltodextrines utilisées conformément à l'invention sont préférentiellement de D.E. allant de 12 à 20.

On utilisera de préférence les maltodextrines de pomme de terre ou de maïs telles que celles vendues sous les dénominations commerciales MD 20 P® de AVEBE, Maldex 120®, Maldex 170®, Maldex 190® de Tereos.

Parmi les glucides hydrosolubles conformes à l'invention, on choisi les maltodextrines de D.E. allant de 4 à 20 et encore mieux les maltodextrines de DE allant de 12 à 20.

Le ou les glucides hydrosoluble(s) conformes à l'invention représentent de 10 à 80% en poids, de préférence de 15 à 70% en poids, plus préférentiellement de 20 à 65% en poids, et encore mieux de 40 à 60% en poids par rapport au poids total de l'enveloppe de la particule.

Selon l'invention, l'enveloppe des particules est constituée

- d'au moins un $C_5$-$C_{20}$-alcényl succinate d'amidon et
- d'au moins une maltodextrine de D.E. allant de 4 à 20 et de préférence allant de 12 à 20.

Selon une première variante, l'enveloppe des particules selon l'invention est constituée d'au moins un $C_5$-$C_{20}$-alcényl succinate d'amidon et d'au moins une maltodextrine de D.E. allant de 12 à 20.

Selon une forme particulièrement préférée de l'invention, l'enveloppe des particules à libération d'agent bénéfique est constituée

a) d'au moins un $C_5$-$C_{20}$-alcényl succinate d'amidon dans une quantité allant de 20 à 90% en poids, notamment 30 à 80% en poids, de préférence 40 à 70% et encore mieux 40 à 60% en poids, par rapport au poids total de l'enveloppe de la particule.

b) et d'au moins une maltodextrine de D.E. allant de 4 à 20 dans une quantité allant de 10 à 80% en poids, de préférence de 15 à 70% en poids, plus préférentiellement de 20 à 65% en poids, et encore mieux de 40 à 60% en poids par rapport au poids total de l'enveloppe de la particule.

## Procédé de préparation des particules d'encapsulation

Les particules selon l'invention peuvent notamment être préparées selon le procédé décrit dans le brevet EP1917098B1 de FeyeCon.

Selon une forme particulière de l'invention, les particules sont obtenues selon un procédé comprenant au moins les étapes suivantes :

- on prépare une solution aqueuse constituée du mélange du glucide hydrosoluble choisi parmi les maltodextrines de Dextrose Equivalent allant de 4 à 20 et du polysaccharide modifié hydrophobe choisi parmi les $C_5$-$C_{20}$-alcényl succinate d'amidon puis on ajoute l'agent bénéfique et on agite de façon à former une émulsion; et
- on homogénéise ladite émulsion ainsi formée sous haute pression à une pression allant de 10 à 200 bars et plus préférentiellement de 20 à 200 bars; et
- on pulvérise, de préférence en continu, ladite émulsion dans une chambre de séchage; et
- l'eau est extraite pendant une durée, de préférence, ne dépassant pas 3 heures, et plus préférentiellement ne dépassant pas 30 minutes, avec un fluide sous pression tel que le dioxyde de carbone, de préférence à l'état supercritique, de préférence à une pression d'au moins 0,3XPc et à une température d'au moins Tc-60°C avec Pc correspondant à la pression critique du gaz et Tc la température critique du gaz, de façon à obtenir des particules, de préférence sphériques, de taille moyenne, de préférence allant de 1 à 150$\mu$m, plus préférentiellement allant de 2 à 100$\mu$m et encore mieux de 5 à 80$\mu$m.

## AGENT STRUCTURANT

Les compositions selon l'invention comprennent au moins un agent structurant qui peut être choisi de préférence parmi les cires, les composés pâteux, les gélifiants lipophiles minéraux ou organiques et leurs mélanges.

Il est entendu que la quantité en ces composés peut être ajustée par l'homme du métier de manière à ne pas porter préjudice à l'effet recherché dans le cadre de la présente invention.

## Corps gras pâteux

Par "corps gras pâteux" (également appelé corps gras pâteux) au sens de la présente invention, on entend un composé gras lipophile à changement d'état solide/liquide réversible, présentant à l'état solide une organisation cristalline anisotrope, et comportant à la température de 23°C une fraction liquide et une fraction solide.

En d'autres termes, la température de fusion commençante du composé pâteux peut être inférieure à 23°C. La fraction liquide du composé pâteux mesurée à 23°C peut représenter 9 à 97% en poids du composé. Cette fraction liquide à 23°C représente de préférence entre 15 et 85%, de préférence encore entre 40 et 85% en poids.

Au sens de l'invention, la température de fusion correspond à la température du pic le plus endothermique observé en analyse thermique (DSC) telle que décrite dans la norme ISO 11357-3; 1999. Le point de fusion d'un pâteux ou d'une cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant:
Un échantillon de 5mg de pâteux ou de cire (selon le cas) disposé dans un creuset est soumis à une première montée en température allant de -20°C à 100°C, à la vitesse de chauffe de 10°C/minute, puis est refroidi de 100°C à -20°C à une vitesse de refroidissement de 10°C/minute et enfin soumis à une deuxième montée en température allant de -20°C à 100°C à une vitesse de chauffe de 5°C/minute. Pendant la deuxième montée en température, on mesure la variation de la différence de puissance absorbée par le creuset vide et par le creuset contenant l'échantillon de pâteux ou de cire en fonction de la température. Le point de fusion du composé est la valeur de la température correspondant au sommet du pic de la courbe représentant la variation de la différence de puissance absorbée en fonction de la température.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (DSC), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 50 à 100%, de préférence encore de 60 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux est de préférence choisi parmi les composés synthétiques et les composés d'origine végétale. Un composé pâteux peut être obtenu par synthèse à partir de produits de départ d'origine végétale.

Le composé pâteux est avantageusement choisi parmi

- la lanoline et ses dérivés
- les éthers de polyol choisi parmi les éthers de pentaérythritol et de polyalkylène glycol, les éthers d'alcool gras et de sucre, et leurs mélanges. l'éther pentaérythritol et de polyéthylène glycol comportant 5 motifs oxyéthylénés (5 OE) (nom CTFA: PEG-5 Pentaérythrityl Ether), l'éther de pentaérythritol et de polypropylène glycol comportant 5 motifs oxypropylénés (5 OP) (nom CTFA : PPG-5 Pentaérythrityl Ether), et leurs mélanges et plus spécialement le mélange PEG-5 Pentaérythrityl Ether, PPG-5 Pentaérythrityl Ether et huile de soja, commercialisé sous la dénomination « Lanolide » par la société Vevy, mélange où les constituants se trouvent dans un rapport en poids 46/46/8 correspondant à 46 % de PEG-5 Pentaérythrityl Ether, 46 % de PPG-5 Pentaérythrityl Ether et 8 % d'huile de soja.
- les composés siliconés polymères ou non
- les composés fluorés polymères ou non
- les polymères vinyliques, notamment:
- les homopolymères et les copolymères d'oléfines
- les homopolymères et copolymères de diènes hydrogénés
- les oligomères linéaires ou ramifiés, homo ou copolymères de (méth)acrylates d'alkyles ayant de préférence un groupement alkyle en $C_8$-$C_{30}$
- les oligomères homo et copolymères d'esters vinyliques ayant des groupements alkyles en $C_8$-$C_{30}$
- les oligomères homo et copolymères de vinyléthers ayant des groupements alkyles en $C_8$-$C_{30}$,
- les polyéthers liposolubles résultant de la polyéthérification entre un ou plusieurs diols en $C_2$-$C_{100}$, de préférence en $C_2$-$C_{50}$,
- les esters,

et/ou leurs mélanges.

Le composé pâteux est de préférence un polymère, notamment hydrocarboné.

Parmi les polyéthers liposolubles, on préfère en particulier les copolymères d'éthylène-oxyde et/ou de propylène-oxyde avec des alkylènes-oxydes à longue chaîne en $C_6$-$C_{30}$, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les esters, on préfère notamment :

- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol
- le propionate d'arachidyle commercialisé sous la marque Waxenol 801 par Alzo,
- les esters de phytostérol,
- les triglycérides d'acides gras et leurs dérivés
- les esters de pentaérythritol,
- les polyesters non réticulés résultant de la polycondensation entre un acide dicarboxylique ou un polyacide carboxylique linéaire ou ramifié en $C_4$-$C_{50}$ et un diol ou un polyol en $C_2$-$C_{50}$,
- les esters aliphatiques d'ester résultant de l'estérification d'un ester d'acide hydroxycarboxylique aliphatique par un acide carboxylique aliphatique. De préférence, l'acide carboxylique aliphatique comprend de 4 à 30 et de préférence de 8 à 30 atomes de carbone. Il est de préférence choisi parmi l'acide héxanoïque, l'acide heptanoïque, l'acide octanoïque, l'acide éthyl-2 héxanoïque, l'acide nonanoïque, l'acide décanoïque, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges. L'acide carboxylique aliphatique est de préférence ramifié. L'ester d'acide hydroxy carboxylique aliphatique est avantageusement issu d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle.

L'ester d'acide hydroxy carboxylique aliphatique est choisi parmi :

a) les esters partiels ou totaux d'acides monocarboxyliques aliphatiques mono hydroxylés linéaires, saturés;
b) les esters partiels ou totaux d'acides monocarboxyliques aliphatiques mono hydroxylés insaturés;
c) les esters partiels ou totaux de polyacides carboxyliques aliphatiques mono hydroxylés saturés;
d) les esters partiels ou totaux de polyacides carboxyliques aliphatiques poly hydroxylés saturés;
e) les esters partiels ou totaux de polyols aliphatiques en $C_2$ à $C_{16}$ ayant réagi avec un mono ou un poly acide carboxylique aliphatique mono ou poly hydroxylé,

et leurs mélanges.

- les esters de dimère diol et dimère diacide, le cas échéant, estérifiés sur leur(s) fonction(s) alcool(s) ou acide(s) libre(s) par des radicaux acides ou alcools, notamment les esters dimer dilinoleate, de tels esters peuvent être notamment choisis parmi les esters de nomenclature INCI suivante: le bis-béhényl/isostéaryl/phytostéryl dimerdilinoléyle dimerdilinoléate (Plandool G), le phytostéryl/isostéryl/cétyl/stéaryl/béhényl dimerdilinoléate (Plandool H ou Plandool S), et leurs mélanges.
- les esters de rosinate hydrogénée, tel que les dimères dilinoleyl de rosinate hydrogéné (Lusplan DD-DHR ou DD-DHR de Nippon Fine Chemical)

et leurs mélanges.

## Cire(s)

Selon un mode de réalisation préféré, la composition selon l'invention comprend au moins une cire.
La cire considérée dans le cadre de la présente invention est d'une manière générale un composé lipophile, solide à température ambiante (25°C), à changement d'état solide/liquide réversible, ayant un point de fusion supérieur ou égal à 30°C pouvant aller jusqu'à 200°C et notamment jusqu'à 120°C.
En particulier, les cires convenant à l'invention peuvent présenter un point de fusion supérieur ou égal à 45°C, et en particulier supérieur ou égal à 55°C.
Les cires susceptibles d'être utilisées dans les compositions selon l'invention sont choisies parmi les cires, solides, à température ambiante d'origine animale, végétale, minérale ou de synthèse et leurs mélanges.
On peut citer par exemple les cires suivantes hydrocarbonées comprenant un chaine alkyle grasse ayant en général de 10 à 60 atomes de carbone, de préférence de 20 à 40 atomes de carbone, ladite chaîne pouvant être saturée ou insaturée, substituée ou non, linéaire, ramifiée ou cyclique, de préférence saturée et linéaire

- les alcools gras solides à température ambiante (25°C) comme l'alcool stéarylique, l'alcool cétylique ou leurs mélanges,
- les esters d'alcools gras,
- les acides gras,
- les amides d'acides gras,
- les esters d'acides gras incluant les triglycérides,
- les éthers d'acides gras,
- les alcools gras éthoxylés,
- les acides gras éthoxylés et leurs sels correspondants.

Parmi les alcools gras, on peut citer l'alcool stéarylique, cétéarylique ou leurs mélanges.
Parmi les esters d'alcools gras, on peut citer tri-isostearyl citrate, ethyleneglycol-di-12-hydroxystearate, tristearyl citrate, stearyl octanoate, hexyldecyl stearate, stearyl heptanoate, tri lauryl citrate et leurs mélanges.
Parmi les esters d'acides gras, on peut citer les cires esters, les monoglycérides, les diglycérides, les triglycérides.

EP 3 166 580 B1

Comme cire ester, on peut citer le stearyl stéarate, le stearyl behenate, le stéaryl octyldodécanol, le cétéaryl béhénate, le béhényl béhénate, l'éthyleneglycol distéarate, l'éthylèneglycol dimaplimitate. On pourra utiliser en particulier un (hydroxystéaryloxy)stéarate d'alkyle en C20-C40 (le groupe alkyle comprenant de 20 à 40 atomes de carbone), seul ou en mélange. Une telle cire est notamment vendue sous les dénominations «Kester Wax K 82 P®», «Hydroxypolyester K 82 P®» et «Kester Wax K 80 P®» par la société KOSTER KEUNEN.

Parmi les cires triglycérides on peut citer plus particulièrement la Tribéhénine, la $C_{18}$-$C_{36}$ Triglycéride et leurs mélanges.

A titre illustratif des cires convenant à l'invention, on peut notamment citer les cires hydrocarbonées comme la cire d'abeille, la cire de lanoline, et les cires d'insectes de Chine, la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en $C_8$-$C_{32}$. Parmi celles-ci, on peut notamment citer l'huile de jojoba isomérisée telle que l'huile de jojoba partiellement hydrogénée isomérisée trans fabriquée ou commercialisée par la société Desert Whale sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.

On peut encore citer les cires de silicone ($C_{30}$-$_{45}$ ALKYL DIMETHICONE), les cires fluorées.

On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A-2792190.

Comme micro cires pouvant être utilisées dans les compositions selon l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 114S® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200®, 220®, 220L® et 250S® par la société MICRO POWDERS et les micro cires de polytétrafluoroéthylène telles que celles commercialisées sous les dénominations de Microslip 519® et 519 L® par la société MICRO POWDERS.

**Gélifiants lipophiles**

Gélifiants minéraux

Comme gélifiant lipophile minéral, on peut citer les argiles éventuellement modifiées comme les hectorites modifiées par un chlorure d'ammonium en $C_{10}$ à $C_{22}$, comme l'hectorite modifiée par du chlorure de di-stéaryl di-méthyl ammonium telle que, par exemple, celle commercialisée sous la dénomination de Bentone 38V® par la société ELEMENTIS.

On peut également citer la silice pyrogénée éventuellement traitée hydrophobe en surface dont la taille des particules est inférieure à 1μm. Il est en effet possible de modifier chimiquement la surface de la silice, par réaction chimique générant une diminution du nombre de groupes silanol présents à la surface de la silice. On peut notamment substituer des groupes silanol par des groupements hydrophobes: on obtient alors une silice hydrophobe. Les groupements hydrophobes peuvent être des groupements trimethylsiloxyle, qui sont notamment obtenus par traitement de silice pyrogénée en présence de l'hexaméthyldisilazane. Des silices ainsi traitées sont dénommées «Silica silylate» selon le CTFA (8ème édition, 2000). Elles sont par exemple commercialisées sous les références Aerosil R812® par la société DEGUSSA, CAB-O-SIL TS-530® par la société CABOT, des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées «Silica diméthyl silylate» selon le CTFA (8ème édition, 2000). Elles sont par exemple commercialisées sous les références Aerosil R972®, et Aerosil R974® par la société DEGUSSA, CAB-O-SIL TS-610® et CAB-O-SIL TS-720® par la société CABOT.

La silice pyrogénée hydrophobe présente en particulier une taille de particules pouvant être nanométrique à micrométrique, par exemple allant d'environ de 5 à 200nm.

## Gélifiants organiques

Les gélifiants lipophiles organiques polymériques sont par exemple les organopolysiloxanes élastomériques partiellement ou totalement réticulés, de structure tridimensionnelle, comme ceux commercialisés sous les dénominations de KSG6®, KSG16® et de KSG18® par la société SHIN-ETSU, de Trefil E-505C® et Trefil E-506C® par la société DOW-CORNING, de Gransil SR-CYC®, SR DMF10®, SR-DC556®, SR 5CYC gel®, SR DMF 10 gel® et de SR DC 556 gel® par la société GRANT INDUSTRIES, de SF 1204® et de JK 113® par la société GENERAL ELECTRIC ; l'éthylcellulose comme celle vendue sous la dénomination Ethocel® par la société DOW CHEMICAL; les galactommananes comportant de un à six, et en particulier de deux à quatre, groupes hydroxyle par ose, substitués par une chaîne alkyle saturée ou non, comme la gomme de guar alkylée par des chaînes alkyle en C1 à C6, et en particulier en C1 à C3 et leurs mélanges. Les copolymères séquencés de type «dibloc», «tribloc» ou «radial» du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton® par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel® comme par exemple le mélange de copolymère tribloc butylène/éthylène/styrène et de copolymère étoile éthylène/propylène/styrène dans l'isododécane (Versagel M 5960).

Comme gélifiant lipophile, on peut encore citer les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, telles que décrites dans les demandes WO-A-02/056847, WO-A-02/47619 dont le contenu est incorporé à titre de référence; en particulier les résines de polyamides (notamment comprenant des groupes alkyles ayant de 12 à 22 atomes de carbone) telles que celles décrites dans US-A-5783657 dont le contenu est incorporé à titre de référence. On peut citer en particulier un mélange de copolymères d'un diacide en C36 condensé sur l'éthylène diamine ; les groupes ester terminaux résultent de l'estérification des terminaisons d'acide restantes par l'alcool cétylique, stéarylique ou leurs mélanges (appelés aussi alcool cétylstéarylique) (nom INCI: ETHYLENEDIAMINE/STEARYL DIMER DILINOLEATE COPOLYMER). Sa masse moléculaire moyenne en poids est de préférence de 6000. Ces mélanges sont notamment vendus par la société Arizona Chemical sous les noms commerciaux Uniclear 80 et Uniclear 100 VG respectivement sous forme de gel à 80% (en matière active) dans une huile minérale et à 100% (en matière active). Ils ont un point de ramollissement de 88°C à 94°C.

Parmi les gélifiants lipophiles pouvant être utilisés dans les compositions selon l'invention, on peut encore citer les polymères issus d'un monomère à chaîne cristallisable choisi parmi les (méth)acrylates d'alkyle saturés en $C_{14}$ à $C_{22}$ et encore plus particulièrement les poly(acrylate de stéaryle) ou les poly(acrylate de béhényle). A titre d'exemple, on peut citer les polymères ayant pour nom INCI " POLY C10-30 ALKYL ACRYLATE comme les produits Intelimer® de la société AIR PRODUCTS comme le produit Intelimer® IPA 13-1 qui est un polyacrylate de stéaryle ou le produit Intelimer® IPA 13-6 qui est un polymère de béhényle.

Parmi les gélifiants lipophiles pouvant être utilisés dans les compositions selon l'invention, on peut encore citer les esters de dextrine et d'acide gras, tels que les palmitates de dextrine, notamment tels que ceux commercialisés sous les dénominations Rheopearl TL® ou Rheopearl KL® par la société CHIBA FLOUR.

On peut également utiliser les polyamides siliconés du type polyorganosiloxane tels que ceux décrits dans les documents US-A-5,874,069, US-A-5,919,441, US-A-6,051,216 et US-A-5,981,680.

Ces polymères siliconés peuvent appartenir aux deux familles suivantes :

-   des polyorganosiloxanes comportant au moins deux groupes capables d'établir des interactions hydrogène, ces deux groupes étant situés dans la chaîne du polymère, et/ou
-   des polyorganosiloxanes comportant au moins deux groupes capables d'établir des interactions hydrogène, ces deux groupes étant situés sur des greffons ou ramifications

Parmi les agents structurants, on choisira plus préférentiellement les pâteux, les cires et gélifiant organiques tels que la cire d'abeille, la cire de lanoline, la cire de Carnauba, la cire de Candellila, les cires d'orange et de citron, les cires microcristallines, les paraffines, l'ozokérite, les cires de polyéthylène, les triglycérides d'acides gras, l'alcool stéarylique, l'alcool cétéarylique, l'alcool cétylstéarylique, l'huile de jojoba isomérisée, l'huile de tournesol hydrogénée, l'huile de ricin hydrogénée, l'huile de coprah hydrogénée, l'hexyldecyl stearate, un mélange de stérols de soja et de pentaérythritol oxyéthyléné (5OE) oxypropyléné (5 OP) et leurs mélanges.

Et en encore plus préférentiellement on choisira les agents structurants choisis parmi l'alcool cétylstéarylique, les cires de Carnauba, la cire de Candellila, les cires de polyéthylène, l'huile de ricin hydrogénée le mélange de stérols de soja et de pentaérythritol oxyéthyléné (5OE) oxypropyléné (5 OP), commercialisé sous la référence Lanolide par la société VEVY, l'ozokérite et l'hexyldecyl stearate et leurs mélanges.

Le ou les agents structurants sont présents dans des quantités allant de préférence de 1 à 70% et plus particulièrement de 4 à 60% par rapport au poids total de la composition et encore plus préférentiellement de 8 à 50%.

## PHASE GRASSE

Selon une forme particulière de l'invention, la composition comprend au moins une phase grasse, notamment à raison de 15 % à 95% en poids et plus particulièrement de 30% à 85 % en poids par rapport au poids total de la composition.

La phase grasse comprend de préférence au moins un composé choisi parmi les huiles, les cires et/ou les solvants d'origine minérale, animale, végétale ou synthétique, carbonés, hydrocarbonés, fluorés et/ou siliconés, volatils ou non volatils, seuls ou en mélange dans la mesure où ils forment un mélange homogène et stable et sont compatibles avec l'utilisation envisagée.

Par 'volatil', on entend au sens de l'invention, tout composé susceptible de s'évaporer au contact des matières kératiniques en moins d'une heure, à température ambiante (25°C) et pression atmosphérique (1atm). Notamment, ce composé volatil a une pression de vapeur non nulle, à température ambiante et pression atmosphérique, notamment allant de 0,13Pa à 40 000Pa ($10^{-3}$ à 300mm de Hg), en particulier allant de 1,3Pa à 13 000Pa (0,01 à 100mm de Hg), et plus particulièrement allant de 1,3Pa à 1300Pa (0,01 à 10mm de Hg).

Par opposition, on entend par 'non volatil', un composé restant sur les matières kératiniques humaines à température ambiante et pression atmosphérique, au moins une heure et ayant notamment une pression de vapeur inférieure à $10^{-3}$mm de Hg (0,13Pa).

## FORMES GALENIQUES

Les compositions selon l'invention se présentent sous toutes les formes solides notamment de bâtonnets (sticks), de pains, de pastilles, etc. Elles contiennent à cet égard les ingrédients généralement utilisés dans ce type de produits et bien connus de l'homme de l'art.

## AGENTS BENEFIQUES

La quantité d'agent bénéfique présent dans les particules conformes à l'invention varie de préférence de 0,1 à 80% en poids du poids de la particule, de préférence 1 à 70% en poids, mieux de 10 à 60% et encore mieux de 15 à 50% en poids du poids total de la particule.

Le temps de libération de l'agent bénéfique variera évidemment selon la nature et l'intensité du stimulus.

La durée totale pour libérer l'agent bénéfique pourra être modulée et sera fortement dépendante de la composition, de la teneur en particules, de la nature notamment chimique de l'agent bénéfique et de sa concentration dans les particules (quantité encapsulée dans la particule) et de la nature et de l'intensité du stimulus auquel sera soumise la particule contenant l'agent bénéfique. La libération peut aussi bien être quasi instantanée ou durer plusieurs heures voire plusieurs jours.

Parmi les agents bénéfiques utilisables selon l'invention, on peut citer plus particulièrement

(i) les corps gras;
(ii) les substances parfumantes;
(iii) les principes actifs pharmaceutiques;
(iv) les actifs cosmétiques.

### a) Corps gras

Ils peuvent être choisis dans le groupe comprenant

(i) les huiles naturelles d'origine végétale, animale ou marine,
(ii) les huiles minérales,
(iii) les huiles hydrogénées,
(iv) les huiles de silicone,
(v) les terpènes,
(vi) le squalène,
(vii) les acides gras saturés ou insaturés,
(viii), les esters d'acide gras,

(x) les cires,

(x) les alcools gras,

(xi) les beurres comme le beurre de karité ou le beurre de cacao,

(xii) et leurs mélanges

## b) **Les substances parfumantes**

Par «substance parfumante», on entend tout ingrédient susceptible de dégager une odeur agréable.

Les parfums sont des compositions contenant notamment des matières premières (dénommées généralement ingrédients de parfumerie) qui sont décrites dans S. Arctander, Perfume and Flavor Chemicals (Montclair, N.J., 1969), dans S. Arctander, Perfume and Flavor Materials of Natural Origin (Elizabeth, N.J., 1960) et dans "Flavor and Fragrance Materials - 1991", Allured Publishing Co. Wheaton, III.

Il peut s'agir de produits de synthèse ou de produits naturels, comme des huiles essentielles, des absolus, des résinoïdes, des résines, des concrètes, et/ou des produits synthétiques (hydrocarbures terpéniques ou sesquiterpéniques, alcools, phénols, aldéhydes, cétones, éthers, acides, esters, nitriles, peroxydes, saturés ou insaturés, aliphatiques ou cycliques).

Selon la définition donnée dans la norme internationale ISO 9235 et adoptée par la Commission de la Pharmacopée Européenne, une huile essentielle est un produit odorant généralement de composition complexe, obtenu à partir d'une matière première végétale botaniquement définie, soit par entraînement à la vapeur d'eau, soit par distillation sèche, soit par un procédé mécanique approprié sans chauffage. L'huile essentielle est le plus souvent séparée de la phase aqueuse par un procédé physique n'entraînant pas de changement significatif de la composition.

Parmi les huiles essentielles utilisables selon l'invention, on peut citer celles obtenues à partir des plantes appartenant aux familles botaniques suivantes:

Abiétaceés ou Pinacées: conifères; Amaryllidacées; Anacardiacées; Anonacées: ylang; Apiacées (par exemple les ombellifères): aneth, angélique, coriandre, criste marine, carotte, persil; Aracées; Aristolochiacées; Astéracées: achilée, armoise, camomille, hélichryse; Bétulacées; Brassicacées; Burséracées: encen ; Caryophyllacées; Canellacées; Césalpiniacées: copaïfera (copahu); Chénopodacées; Cistacées: ciste; Cypéracées; Diptérocarpacées; Ericacées: gaulthérie (wintergreen); Euphorbiacées; Fabacées; Geraniacées: géranium; Guttifères; Hamamélidacées; Hernandiacées; Hypéricacées: millepertuis; Iridacées; Juglandacées; Lamiacées: thym, origan, monarde, sarriette, basilic, marjolaines, menthes, patchouli, lavandes, sauges, cataire, romarin, hysope, mélisse; Lauracées: ravensara, laurier, bois de rose, cannelle, litséa; Liliacées: ail, lys, muguet, jacinthe, jonquille,...; Magnoliacées: magnolia; Malvacées ; Méliacées; Monimiacées; Moracées: chanvre, houblon; Myricacées; Mysrticacées: muscade; Myrtacées: eucalyptus, tea tree, niaouli, cajeput, backousia, girofle, myrte; Oléacées; Pipéracées: poivre ; Pittosporacées; Poacées: citronnelle, lemongrass, vétiver; Polygonacées; Renonculacées; Rosacées: roses; Rubiacées; Rutacées: tous les citrus; Salicacées; Santalacées: santal; Saxifragacées; Schisandracées; Styracacées: benjoin; Thymélacées: bois d'agar; Tilliacées; Valérianacées: valériane, nard; Verbénacées: lantana, verveine; Violacées; Zingibéracées: galanga, curcuma, cardamome, gingembre; Zygophyllacées.

On peut citer également les huiles essentielles extraites de fleurs (lys, lavande, rose, jasmin, Ylang-Ylang, néroli), de tiges et de feuilles (patchouli, géranium, petit-grain), de fruits (framboise, pêche ,coriandre, anis, cumin, genièvre), d'écorces de fruits (bergamote, citron, orange, pamplemousse), de racines (angélique, céleri, cardamome, iris, acore, gingembre), de bois (bois de pin, santal, gaïac, cèdre rose, camphre), d'herbes et de graminées (estragon, romarin, basilic, lemon grass, sauge, thym), d'aiguilles et de branches (épicéa, sapin, pin, pin nain), de résines et de baumes ( galbanum, élémi, benjoin, myrrhe, oliban, opopanax).

Des exemples de substances parfumantes sont notamment : le géraniol, l'acétate de géranyle, le farnésol, le bornéol, l'acétate de bornyle, le linalool, l'acétate de linalyle, le propionate de linalyle, le butyrate de linalyle, le tétrahydrolinalool, le citronellol, l'acétate de citronellyle, le formiate de citronellyle, le propionate de citronellyle, le dihydromyrcénol, l'acétate de dihydromyrcényle, le tétrahydromyrcénol, le terpinéol, l'acétate de terpinyle, le nopol, l'acétate de nopyle, le nérol, l'acétate de néryle, le 2-phényléthanol, l'acétate de 2-phényléthyle, l'alcool benzylique, l'acétate de benzyle, le salicylate de benzyle, l'acétate de styrallyle, le benzoate de benzyle, le salicylate d'amyle, le diméthylbenzylcarbinol, l'acétate de trichlorométhylphénylcarbinyle, l'acétate de p-tert-butylcyclohexyle, l'acétate d'isononyle, l'acétate de cis-3-hexenyle , l'acétate de vétivéryle, l'acétate d'éthyle, l'acétate de butyle, l'acétate d'hexyle, l'acétate de décyle, l'acétate d'isoamyle, l'acétate de stéaryle, l'heptanoate d'allyle, le vétivérol, l'alpha-hexylcinnamaldéhyde, le 2-méthyl-3-(p-tert-butylphényl)propanal, le 2-méthyl-3-(p-isopropylphényl)propanal, le 3-(p-tert-butylphényl)-propanal, le 2,4-diméthylcyclohex-3-enyl-carboxaldéhyde, l'acétate de tricyclodécènyle, le propionate de tricyclodécènyle, l'allyl 3-cyclohexylpropionate, l'éthyl-6-(acétyloxy)-hexanoate, le caproate d'allyle, l'éthyl-2 méthyl butyrate, le méthyl dihydrojasmonate, le salicylate d'hexyle, le 4-(4-hydroxy-4-méthylpentyl)-3-cyclohèxenecarboxaldéhyde, le 4-(4-méthyl-3-pentènyl)-3-cyclohèxenecarboxaldéhyde, le 4-acétoxy-3-pentyl-tétrahydropyrane, le 3-carboxyméthyl-2-pentylcyclopentane, la 2-n-4-heptylcyclopentanone, la 3-méthyl-2-pentyl-2-cyclopentènone, la menthone, la carvone, la tagétone, la géranyl acétone, le n-décanal, le n-dodécanal, l'anisyl propanal, le 9-décènol-1, le cis-3-hexénol, le tétrahydro-2-isobutyl-4-méthylpyrann-4-ol, 3-Methyl-5-phényl-1-pentanol, le 3a,6,6,9a-Tétraméthyl-dodécahydronaphtho[2,1-b]furanne, l'isobutyrate de phénoxyéthyle, le phényl-acétaldéhyde diméthyl-acétal, le phénylacétaldéhyde diéthylacétal, le géranonitrile, le citronellonitrile, l'acétate de cédryle, le 3-isocamphylcyclohexanol, le cédryl méthyl éther, l'isolongifolanone, l'aubépinonitrile, l'aubépine, l'héliotropine, la coumarine, l'eugénol, la vanilline, l'oxyde de diphényle, le citral, le citronellal, l'hydroxycitronellal, l'hexylcinnamal, le 2,4-dimethylcyclohex-3-ène-1-carbaldéhyde, le 2,6-dimethylhept-5-ènal, l'α,α-diméthyl-p-éthylphénylpropanal, le 1,3-benzodioxole-5-carboxaldéhyde, le limonène, la damascone, la décalactone, la nonalactone, le 6,6-diméthoxy-2,5,5-trimethylhex-2-ène, la 2,4,4,7-tétraméthyloct-6-èn-3-one, la1-(5,5-dimethyl-1-cyclohexenyl)-pent-4-èn-1-one, la méthylheptènone, le 4-(cyclopropylmethyl)-phénylméthyl éther, le 2-methyl-6-methylideneoct-7-en-2-ol, l'oxyde de rose, la 1-(1,2,3,4,5,6,7,8-octahydro-2,3,8,8-tetraméethyl-2-naphthyl)éthan-1-one, la 2-acétonaphthone, la 2-isopropyl-5-méthylcyclohexanone, les ionones, les méthylionones, les isométhylionones, la solanone, les irones, le cis-3-hexénol et ses esters, les muscs-indanes, les muscs-tétralines, les muscs-isochromanes, les cétones macrocycliques, les muscs-macrolactones, les muscs aliphatiques, le brassylate d'éthylène, l'essence de rose et leurs mélanges.

D'une façon générale, les parfums sont constitués d'un mélange d'ingrédients dits de parfumerie qui peuvent être également classés en notes de tête, notes de cœur et notes de fond.

Les trois notes correspondent à la volatilité plus ou moins importante des ingrédients qui les composent: note de tête fortement volatile, note de coeur moyennement volatile et note de fond faiblement volatile.

(i) La note de tête appelée aussi «départ» est celle que l'odorat perçoit en premier dès que le parfum est en contact avec la matière kératinique ou tout substrat. Mais c'est celle qui s'atténue le plus rapidement: elle ne "tient pas". Il est difficile d'exprimer le temps de persistance de cette note, car il est très variable: de quelques minutes à une dizaine de minutes

Elle est essentiellement fraîche et légère. Tous les agrumes appartiennent notamment à cette catégorie. En parfumerie, on les range sous le terme générique d'hespéridés dont font partie orange, citron, pamplemousse, bergamote, néroli etc... On citera également les aromates tels que la lavande, le laurier, le thym ou le romarin et les anisés, mentholés, aldéhydés, etc. On citera également les notes eucalyptus.

(ii) La note de cœur, parfois appelée aussi «corps» a une persistance qui va de quelques dizaines de minutes à quelques heures, mais sa principale caractéristique est de ne se révéler qu'au bout de quelques minutes. Elle "démarre" donc juste avant l'extinction de la note de tête. Elle commence à s'exprimer alors que la note de tête s'efface progressivement. Elle est représentée essentiellement par des éléments floraux, fruités ou épicés: muguet, chèvrefeuille, violette, magnolia, cannelle, géranium, jasmin, rose, iris, framboise, pêche, etc...

(ii) La note de fond, parfois appelée aussi «fond» assure la "durabilité", la persistance ou la ténacité d'un parfum. Elle est perceptible plusieurs heures, voire plusieurs jours, ou même plusieurs semaines après application. sur un vêtement ou sur une mouillette ou touche olfactive, selon la concentration du parfum. On citera par exemple les bois, racines, mousses, résines et les substances animales ou minérales telles que opoponax, muscs, ambre, santal, benjoin, lichen, clou de girofle, sauge, etc. On citera également les notes vanillées, le patchouli, les coumarines...etc.

On peut bien entendu encapsuler des ingrédients appartenant à une ou plusieurs notes. Toutefois, on préférera encapsuler les ingrédients les plus volatiles (= les moins rémanents) appartenant aux notes de tête et/ou de cœur. Parmi ces ingrédients, on citera, par exemple :

Acétate de benzyle
Acétate de géranyle
Acétate de cis-3-hexenyle
Aldéhyde C18 ou nonalactone
Acétate de décyle
Allyl amyl glycolate (citral)
Acétate d'éthyle
Acétate de butyle
Allyl 3-cyclohexylpropionate
Acétate de linalyle
Alcool Phényléthylique
Acétate d'héxyle

Berryflor ou éthyl-6-(acétyloxy)-hexanoate
Acétate d'isoamyle
Caproate d'allyle
Amarocite ou 6,6-diméthoxy-2,5,5-triméthylhex-2-ène
Citral lemarome N ou 3,7-diméthylocta-2,6-diénal
Canthoxal ou anisyl propanal
Claritone ou 2,4,4,7-tétraméthyloct-6-èn-3-one
Ethyl-2méthyl butyrate
Dihydromyrcénol
Cis-3 hexenol
Hédione ou méthyl dihydrojasmonate
L-carvone
Heptanoate d'allyle
Limonène
Néobuténone alpha ou 1-(5,5-diméthyl-1-cyclohexenyl)- pent-4-èn-1-one
Méthylheptènone
Toscanol ou 4-(cyclopropylméthyl)-phénylméthyl éther
myrcenol super ou 2-methyl-6-methylideneoct-7-en-2-ol
Decalactone
Acétate de stéaryle
Oxyde de rose
Linalool
Triplai ou 2,4-dimethylcyclohex-3-ène-1-carbaldéhyde
Mélonal ou 2,6-dimethylhept-5-ènal
1-(1,2,3,4,5,6,7,8-octahydro-2,3,8,8-tetraméethyl-2-naphthyl)éthan-1-one
Hexylcinnamal
Tétrahydro-2-isobutyl-4-méthylpyrann-4-ol
Salicylate d'hexyle
1,4-Dioxacycloheptadécane-5, 17-dione
et leurs mélanges

Selon une forme particulière de l'invention, les particules d'encapsulation comprennent au moins une ou plusieurs substances parfumantes ayant une pression de vapeur saturante à 25°C supérieure ou égale à 10,0Pa

La pression de vapeur saturante (ou tension de vapeur) est la pression à laquelle la phase gazeuse d'une substance est en équilibre avec sa phase liquide ou solide à une température donnée dans un système fermé. Le calcul de la pression de vapeur saturante peut se faire à l'aide de la formule suivante :

$$\ln \frac{p_{\text{sat}}}{p_0} = \frac{M.L_v}{R} \left( \frac{1}{T_0} - \frac{1}{T} \right)$$

avec :

- $T_0$: température d'ébullition de la substance à une pression $p_0$ donnée, en degrés Kelvin,
- $p_{\text{sat}}$: pression de vapeur saturante, dans la même unité que po
- M: masse molaire de la substance, en kg/mol
- $L_v$: chaleur latente de vaporisation de la substance, en Joules/kg
- R: constante des gaz parfaits, égale à 8,31447J/K/mol
- T: température de la vapeur, en K.

De préférence les substances parfumantes ayant une pression de vapeur saturante à 25°C supérieure ou égale à 10Pa représentent une quantité allant de 50 à 100% en poids, de préférence de 60 à 100% en poids, plus préférentiellement de 70 à 100% en poids, et encore mieux de 80 à 100% en poids par rapport au poids total des substances parfumantes présentes dans les particules de l'invention.

## c) Les principes actifs pharmaceutiques

On entend par «principe actif pharmaceutique» une molécule ou un mélange de molécules qui possède un effet thérapeutique, curatif et/ou prophylactique pouvant être administré par pulvérisation.

## d) Les actifs cosmétiques

On entend par «actif cosmétique» toute molécule qui possède un effet d'hygiène, de soin, de maquillage, de coloration contribuant à l'amélioration, du bien-être et/ou à l'embellissement ou la modification de l'aspect de la matière kératinique humaine sur laquelle on applique ladite composition.

Parmi les actifs cosmétiques susceptibles d'être appliqués sur des matières kératiniques humaines telles que la peau, les lèvres, le cuir chevelu, les cheveux, les cils ou les ongles, on peut citer par exemple, seuls ou en mélanges :

- les vitamines et leurs dérivés ou précurseurs, seuls ou en mélanges,
- les agents anti-oxydants;
- les agents nettoyants tels que les tensio-actifs;
- les matières colorantes;
- les agents conditionneurs,
- les agents pour le défrisage et/ou le lissage et/ou la mise en forme des cheveux ;
- les agents anti-radicalaires;
- les agents photoprotecteurs comme les filtres UV organiques ou inorganiques,
- les agents autobronzants,
- les agents anti-glycation;
- les agents apaisants,
- les agents dépilatoires,
- les agents déodorants,
- les agents anti-transpirants,
- les inhibiteurs de NO-synthase;
- les agents stimulant la prolifération des fibroblastes;
- les agents stimulant la prolifération des kératinocytes;
- les agents dermorelaxants,
- les agents rafraîchissants,
- les agents tenseurs,
- les agents matifiants,
- les agents anti-luisance de la peau,
- les agents anti-séborrhéiques,
- les agents anti-cheveux gras,
- les agents dépigmentants,
- les agents pro-pigmentants
- les agents kératolytiques,
- les agents desquamants;
- les agents hydratants,
- les agents anti-microbiens,
- les agents amincissants,
- les agents agissant sur le métabolisme énergétique des cellules,
- les agents répulsifs contre les insectes,
- les antagonistes de substances P ou de CRGP ;
- les agents anti-chute des cheveux ;
- les agents anti-rides,
- les agents anti-vieillissement ;
- les agents antipelliculaires.

Parmi ces actifs cosmétiques, on préférera tout particulièrement, seuls ou en mélanges

- les agents photoprotecteurs comme les filtres UV en particulier les filtres UV organiques;
- les agents anti-luisance de la peau,
- les agents anti-séborrhéiques,
- les agents anti-cheveux gras,

- les agents déodorants,
- les agents anti-transpirants,
- les agents rafraîchissants,
- les agents matifiants,
- les agents anti-microbiens,
- les agents antipelliculaires.

Selon une forme particulièrement préférée de l'invention, le ou les agents bénéfiques présents dans les particules seront choisis parmi les substances parfumantes.

Selon une forme particulière de l'invention, la composition contiendra :

a) des particules renfermant au moins une substance parfumante et
b) au moins une substance parfumante sous forme libre, identique ou différente de la substance parfumante présente dans les dites particules.

Lesdites substances parfumantes sous forme libre peuvent être choisies parmi celles citées précédemment.

Selon une autre forme particulière de l'invention, la composition contient exclusivement la ou les substances parfumantes dans les particules d'encapsulation. Autrement dit, la totalité des ingrédients pour parfumer présents dans la composition sont contenus dans les particules.

L'homme du métier pourra choisir la composition appropriée, ainsi que sa méthode de préparation, sur la base de ses connaissances générales, en tenant compte d'une part de la nature des constituants utilisés et d'autre part de l'application envisagée pour la composition.

Selon une forme particulière de l'invention, les compositions selon l'invention sont des produits de maquillage où la composition comprend au moins un agent de coloration sous forme libre et/ou sous forme encapsulée notamment choisi parmi les nacres, les pigments, les particules réfléchissantes, et leur(s) mélange(s). Ces produits peuvent être notamment des rouge à lèvres, des fonds de teint coulés en stick ou en coupelle, des fards à joues ou paupières, des stick anti-cernes, des brillants à lèvres (gloss), des eye-liners, des mascaras. Plus particulièrement, les particules à libération comprennent au moins une substance parfumante. Encore plus particulièrement, les compositions contiendront en plus une substance parfumante sous forme libre, identique ou différente de la substance parfumante présente dans les particules.

## Agents de coloration

Selon une forme particulière de l'invention, la phase pulvérulente comprend avantageusement au moins un agent de coloration. Cette phase pulvérulente comprend de préférence plus largement au moins un agent de coloration choisi parmi las nacres, les pigments, les particules réfléchissantes, et leur(s) mélange(s).

Ladite composition présente avantageusement une teneur en agents de coloration, et en particulier en pigment(s), supérieure ou égale à 0.01% en poids par rapport au poids total de la composition.

### Pigments

Par «pigments», il faut comprendre des particules de toute forme, blanches ou colorées, minérales ou organiques, insolubles dans le milieu physiologique, destinées à colorer la composition.
Les pigments peuvent être blancs ou colorés, minéraux et/ou organiques.

On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Les pigments organiques peuvent être choisis parmi les matériaux ci-dessous et leurs mélanges:

le carmin de cochenille,
les pigments organiques de colorants azoïques, anthraquinoniques, indigoïdes, xanthéniques, pyréniques, quinoliniques, de triphénylméthane, de fluorane.

Parmi les pigments organiques, on peut notamment citer les pigments certifiés D&C connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

Les matériaux chimiques correspondant à chacune des matières colorantes organiques citées précédemment sont mentionnés dans l'ouvrage «International Cosmetic Ingredient Dictionnary and Handbook», Edition 1997, pages 371 à 386 et 524 à 528, publié par «The Cosmetic, Toiletry, and Fragrance Association», dont le contenu est incorporé dans la présente demande par référence.

Une composition selon l'invention peut comprendre une teneur en pigments allant de 0 % à 30% en poids, par rapport au poids total de la composition, de préférence allant de 2 % à 20% en poids, et préférentiellement allant de 4 % à 10% en poids par rapport au poids total de la composition.

Nacres

Par «nacres», il faut comprendre des particules colorées de toute forme, irisées ou non, notamment produites par certains mollusques dans leur coquille ou bien synthétisées et qui présentent un effet de couleur par interférence optique.

Comme exemples de nacres, on peut citer les pigments nacrés tels que le mica titane recouvert avec un oxyde de fer, le mica recouvert d'oxychlorure de bismuth, le mica titane recouvert avec de l'oxyde de chrome, les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être introduites dans la composition, on peut citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona); les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

Toujours à titre d'exemple de nacres, on peut citer également les particules comportant un substrat de borosilicate enrobé d'oxyde de titane.

Des particules à substrat de verre revêtu d'oxyde de titane sont notamment vendues sous la dénomination METASHINE MC1080RY par la société TOYAL.

Enfin, comme exemples de nacres, on peut également citer les paillettes de polyéthylène téréphthalate, notamment celles commercialisées par la société Meadowbrook Inventions sous le nom Silver 1P 0.004X0.004 (paillettes argentées).

Les compositions selon l'invention peuvent comprendre une teneur en nacres allant de 0% à 30% en poids, par exemple de 0.01 à 5% en poids, par rapport au poids total de la composition.

Particules réfléchissantes

Par «particules réfléchissantes», on désigne des particules dont la taille, la structure, notamment l'épaisseur de la ou des couches qui la constituent et leurs natures physique et chimique, et l'état de surface, leur permettent de réfléchir la lumière incidente. Cette réflexion peut, le cas échéant, posséder une intensité suffisante pour créer à la surface de la composition ou du mélange, lorsque celui-ci est appliqué sur le support à maquiller, des points de surbrillance visibles à l'œil nu, c'est-à-dire des points plus lumineux qui contrastent avec leur environnement en semblant briller.

Les particules réfléchissantes peuvent être sélectionnées de manière à ne pas altérer significativement l'effet de coloration généré par les agents de coloration qui leur sont associés et plus particulièrement de manière à optimiser cet effet en terme de rendu de couleur. Elles peuvent plus particulièrement posséder une couleur ou un reflet jaune, rose, rouge, bronze, orangé, brun, or et/ou cuivré.

Ces particules peuvent présenter des formes variées, notamment être en forme de plaquettes ou globulaires, en particulier sphériques. Les particules réfléchissantes, quelle que soit leur forme, peuvent présenter une structure multicouche ou non et, dans le cas d'une structure multicouche, par exemple au moins une couche d'épaisseur uniforme, notamment d'un matériau réfléchissant.

Lorsque les particules réfléchissantes ne présentent pas de structure multicouche, elles peuvent être composées par exemple d'oxydes métalliques, notamment des oxydes de titane ou de fer obtenus par synthèse.

Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant notamment d'au moins un métal ou matériau métallique. Le substrat peut être monomatière, multimatériau, organique et/ou inorganique.

Plus particulièrement, il peut être choisi parmi les verres, les céramiques, le graphite, les oxydes métalliques, les alumines, les silices, les silicates, notamment les aluminosilicates et les borosilicates, le mica synthétique et leurs mélanges, cette liste n'étant pas limitative.

Le matériau réfléchissant peut comporter une couche de métal ou d'un matériau métallique. Des particules réfléchissantes sont décrites notamment dans les documents JP-A-09188830, JP-A-10158450, JP-A-10158541, JP-A-07258460 et JP-A-05017710.

Toujours à titre d'exemple de particules réfléchissantes comportant un substrat minéral enrobé d'une couche de métal, on peut citer également les particules comportant un substrat de borosilicate enrobé d'argent.

Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

On peut également utiliser des particules comprenant un substrat métallique tel que l'argent, l'aluminium, le fer, le chrome, le nickel, le molybdène, l'or, le cuivre, le zinc, l'étain, le magnésium, l'acier, le bronze, le titane, ledit substrat étant enrobé d'au moins une couche d'au moins un oxyde métallique tels que l'oxyde de titane, l'oxyde d'aluminium, l'oxyde de fer, l'oxyde de cérium, l'oxyde de chrome, les oxydes de silicium et leurs mélanges.

On peut citer à titre d'exemple les poudres d'aluminium, de bronze ou de cuivre enrobées de $SiO_2$ commercialisées sous la dénomination VISIONAIRE par la société ECKART.

Les compositions selon l'invention peuvent comprendre une teneur en particules réfléchissantes allant de 0% à 30% en poids, par exemple de 0.01 à 5% en poids, par rapport au poids total de la composition.

De préférence, les agents de coloration sont choisis parmi:

- les pigments organiques avantageusement choisies parmi les pigments certifiés D & C par la Food & Drug Administration tels que répertoriés à la section « Color Additives - Batch Certified by the U.S. Food and Drug Administrationage » du CTFA ; on peut citer notamment les Blue 1 et 4, Brown 1, Ext.Violet 2, Ext.Yellow 7, les Green 3, 5, 6, 8, les Orange 4, 5, 10, 11, les Red 4, 6, 7, 17, 21, 22, 27, 28, 30, 36 et 40, le Violet 2, les Yellow 5, 6, 7, 8, 10 et 11, et leurs mélanges,
- les pigments minéraux avantageusement choisies parmi les oxydes de fer, de titane, de zirconium, de cérium, de zinc, de fer ou de chrome, le bleu ferrique, le violet de manganèse, le bleu, le rose ou le violet d'outremer, l'hydrate de chrome, l'hydroxyde de chrome, l'oxychlorure de bismuth, et leurs mélanges.

Selon une forme particulière de l'invention, les compositions selon l'invention sont des produits de soin de la peau notamment du visage ou des lèvres où la composition comprend au moins un actif cosmétique ou dermatologique. Ces produits peuvent être notamment des baumes ou bases de soin pour les lèvres, des baumes ou bases de soins journaliers ou encore des sticks solaires ou auto-bronzants. Plus particulièrement, les particules à libération comprennent au moins une substance parfumante. Encore plus particulièrement, les compositions contiendront en plus une substance parfumante sous forme libre, identique ou différente de la substance parfumante présente dans les particules.

Selon une autre forme particulière de l'invention, les compositions selon l'invention peuvent se présenter sous forme de produits déodorants et/ou anti-transpirants sous forme libre et/ou encapsulée où la composition comprend au moins un actif déodorant et/ou au moins un actif anti-transpirant. Plus particulièrement, les particules à libération comprennent au moins une substance parfumante. Encore plus particulièrement, les compositions contiendront en plus une substance parfumante sous forme libre, identique ou différente de la substance parfumante présente dans les particules.

Ce mode de réalisation présente l'avantage de libérer quasi instantanément après rupture des particules au contact de l'eau ou de la sueur une substance parfumante destinée à apporter un effet fraicheur.

**Actif anti-transpirant**

Par «actif anti-transpirant», on entend un composé I qui, à lui seul, a pour effet de diminuer le flux sudoral, et/ou de diminuer la sensation sur la peau d'humidité liée à la sueur humaine et/ou d'absorber partiellement ou totalement la sueur humaine.

Parmi les actifs antitranspirants, on peut citer les sels d'aluminium et/ou de zirconium tels que le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate et plus particulièrement le chlorhydrate d'aluminium sous forme activée ou non commercialisé par la société REHEIS sous la dénomination MICRODRY ALUMINUM CHLOROHYDRATE® ou par la société GUILINI CHEMIE sous la dénomination ALOXICOLL PF 40. Des sels d'aluminium et de zirconium sont par exemple celui commercialisé par la société REHEIS sous la dénomination REACH AZP-908-SUF®, des sel d'aluminium « activés » par exemple celui commercialisé par la société REHEIS sous la dénomination REACH 103 ou par la société WESTWOOD sous la dénomination WESTCHLOR 200.

De préférence, la composition cosmétique comprend le chlorhydrate d'aluminium en tant qu'actif anti-transpirant.

Comme autre actif anti-transpirant, on peut citer les particules de perlite expansée telles que celles obtenues par le procédé d'expansion décrit dans le brevet US 5,002,698.

Les perlites utilisables selon l'invention sont généralement des aluminosilicates d'origine volcanique et ont comme composition

70,0-75,0% en poids de silice $SiO_2$
12,0-15,0% en poids d'oxyde d'aluminium oxyde $Al_2O_3$
3,0-5,0% d'oxyde de sodium $Na_2O$
3,0-5,0% d'oxyde de potassium $K_2O$
0,5-2% d'oxyde de fer $Fe_2O_3$
0,2-0,7% d'oxyde de magnesium $MgO$
0,5-1,5% d'oxyde de calcium $CaO$
0,05 - 0,15% d'oxyde de titane $TiO_2$

De préférence, les particules de perlite utilisées seront broyées ; elles sont dans ce cas dites Expanded Milled Perlite (EMP). Elles ont de préférence une taille de particule définie par un diamètre médian D50 allant de 0,5 à 50$\mu$m et de préférence de 0,5 à 40$\mu$m.

De préférence, les particules de perlite utilisées présentent une densité apparente non tassée à 25°C allant de 10 et 400kg/m3 (Norme DIN 53468) et de préférence de 10 et 300kg/m$^3$.

De préférence, les particules de perlite expansée selon l'invention ont une capacité d'absorption d'eau mesurée au WET POINT allant de 200 à 1500% et de préférence de 250 à 800%.

Le Wet Point correspond à la quantité d'eau qu'il faut additionner à 100 g de particules pour obtenir une pâte homogène. Cette méthode dérive directement de celle de la prise d'huile appliquée aux solvants. Les mesures sont faites de la même manière par l'intermédiaire du Wet Point et du Flow Point ayant respectivement comme définition suivante :
WET POINT: masse exprimée en grammes pour 100g de produit correspondant à l'obtention d'une pâte homogène lors de l'addition d'un solvant à une poudre.

FLOW POINT : masse exprimée en grammes pour 100g de produit à partir de laquelle la quantité de solvant est supérieure à la capacité de la poudre à le retenir. Cela se traduit par l'obtention d'un mélange plus ou moins homogène s'écoulant sur la plaque de verre.

Le Wet Point et le Flow point sont mesurés selon le protocole suivant:

Protocole de mesure de l'absorption d'eau.

1) Matériel utilisé

Plaque de verre (25 x 25 mm)
Spatule (manche en bois et partie métallique (15 x 2,7mm)
Pinceau à poils de soie
Balance

## 2) Mode Opératoire

On dépose la plaque de verre sur la balance et on pèse 1g de particules de perlite. On dépose le bécher contenant le solvant ainsi que la liquipipette de prélèvement sur la balance. On ajoute progressivement le solvant à la poudre en malaxant régulièrement l'ensemble (toutes les 3 à 4 gouttes) à l'aide de la spatule

On note la masse de solvant nécessaire à l'obtention du Wet Point. On ajoute à nouveau le solvant et on note la masse permettant d'arriver au Flow Point. On effectuera la moyenne sur 3 essais.

On utilisera en particulier les particules de perlite expansée vendues sous les noms commerciaux OPTIMAT 1430 OR ou OPTIMAT 2550 par la société WORLD MINERALS.

## Actifs déodorants

On appelle « actif déodorant », toute substance capable de masquer, absorber, améliorer et/ou réduire l'odeur désagréable résultant de la décomposition de la sueur humaine par des bactéries

Les actifs déodorants peuvent être des agents bactériostatiques ou des agents bactéricides agissant sur les germes des odeurs axillaires, comme le 2,4,4'-trichloro-2'-hydroxydiphényléther (®Triclosan), le 2,4-dichloro-2'-hydroxydiphényléther, le 3',4',5'-trichlorosalicylanilide, la 1-(3',4'-dichlorophenyl)-3-(4'-chlorophenyl)urée (®Triclocarban) ou le 3,7,11-triméthyldodéca-2,5,10-triénol (®Farnesol) ; les sels d'ammonium quaternaires comme les sels de cetyltrimethylammonium, les sels de cétylpyridinium ; les polyols comme ceux de type glycérine, 1,3-propanediol (ZEMEA PROPANEDIOL® commercialisé par Dupont Tate and Lyle Bioproducts), le 1,2-décanediol (SYMCLARIOL® de la société Symrise) ; les dérivés de glycérine comme par exemple le Caprylic/Capric Glycerides (CAPMUL MCM® de Abitec), le Caprylate ou caprate de Glycerol (DERMOSOFT GMCY® et DERMOSOFT GMC® respectivement de STRAETMANS), le Polyglyceryl-2 Caprate (DERMOSOFT DGMC® de STRAETMANS) les dérivés de biguanide comme les sels de polyhexaméthylène biguanide ; la chlorhexidine et ses sels; le 4-Phenyl-4,4-dimethyl-2butanol (SYMDEO MPP® de Symrise) ; les cyclodextrines ; les chélatants tels que le Tetrasodium Glutamate Diacetate (CAS #51981-21-6) vendu sous le nom commercial DISSOLVINE GL-47-S® de Akzo Nobel, l'EDTA (acide Ethylendiamino Tétraacétique) et le DPTA (acide 1,3-diaminopropanetétraacétique).

Parmi les actifs déodorants conformes à l'invention, on peut aussi citer également

- les sels de zinc comme le salicylate de zinc, le phénolsulfonate de zinc, le pyrrolidone carboxylate de zinc (plus communément appelé pidolate de zinc), le sulfate de zinc, le chlorure de zinc, le lactate de zinc, le gluconate de zinc, ricinoléate de zinc, glycinate de zinc, carbonate de zinc, citrate de zinc, chlorure de zinc, le laurate de zinc, l'oléate de zinc, l'orthophosphate de zinc, le stéréate de zinc, le tartrate de zinc, l'acétate de zinc ou leurs mélanges ;
- des absorbeurs d'odeurs comme les zéolites notamment métalliques sans argent, les cyclodextrines, les silicates d'oxyde métallique telles que celles décrite dans la demande US2005/063928 ; des particules d'oxyde métallique modifiées par un métal de transition telles que décrites dans les demandes US2005084464 et US2005084474, des aluminosilicates comme ceux décrits dans la demande EP1658863, des particules de dérivés de chitosan comme celles décrites dans le brevet US6916465 ;
- le bicarbonate de sodium ;
- l'acide salicylique et ses dérivés tels que l'acide n-octanoyl-5-salicylique
- l'alun.
- le triéthyl citrate

Les actifs déodorants peuvent être présents de préférence dans les compositions selon l'invention dans des concentrations pondérales allant 0,01 à 10% en poids par rapport au poids total de la composition.

La présente invention concerne également un procédé cosmétique de traitement des odeurs corporelles et éventuellement de la transpiration humaine consistant à appliquer sur la surface de la matière kératinique une composition telle que définie précédemment comprenant au moins un actif déodorant et/ou un actif anti-transpirant.

L'invention est illustrée plus en détail dans les exemples suivants.

## Exemples de préparation de particules à libération de parfum

## Exemple A

On a préparé des capsules en mettant en œuvre la composition suivante:

| Composition | Amidon modifié hydrophobe | Polysaccharide hydrosoluble | Parfum* | Eau |
|---|---|---|---|---|
| Exemple A | Amidon Capsul® de National Starch 110g | Maltodextrine de pomme de terre MD 20 P de AVEBE 110g | 55g | 225g |

* Le parfum utilisé a la composition suivante :

| Ingrédients | Quantité en g |
|---|---|
| Myristate d'isopropyle | 20,5 |
| Méthyl dihydrojasmonate | 15 |
| 2-phenyléthanol | 8 |
| 1-(1,2,3,4,5,6,7,8-octahydro-2,3,8,8-tetraméthyl-2-naphthyl)éthan-1-one | 8 |

| | |
|---|---|
| Hexylcinnamal | 6 |
| Tétrahydro-2-isobutyl-4-méthylpyrann-4-ol | 6 |
| Hexyl Salicylate | 6 |
| Benzyl Acétate | 5 |
| 1,4-Dioxacycloheptadécane-5,17-dione | 5 |
| 3-Methyl-5-phényl-1-pentanol | 5 |
| dihydromyrcenol | 4 |
| ORANGE TERPENES 0,05% B H T (limonène >95%) | 4 |
| 2-acétonaphthone | 2 |
| 3a,6,6,9a-Tétraméthyl-dodécahydronaphtho[2,1-b]furanne | 1 |
| α,α-diméthyl-p-éthylphénylpropanal | 1 |
| 1,3-benzodioxole-5-carboxaldéhyde | 1 |
| 2-isopropyl-5-méthylcyclohexanone | 1 |
| 1-phényléthyl acetate | 0,8 |
| 2,6-dimethylhept-5-ènal (mélonal) | 0,5 |
| 2,4-dimethylcyclohex-3-ène-1-carbaldehyde (triplal) | 0,2 |

**Procédé de préparation de l'émulsion**

On a mélangé la maltodextrine de pomme de terre MD20 P et l'amidon CAPSUL® dans l'eau jusqu'à dissolution puis on a ajouté le parfum et émulsionné avec un disperseur Ultraturrax de type Heidolph Diax 900 (moteur de puissance 900W avec une vitesse de 8000 à 26000 tour/mn électroniquement contrôlée) à la puissance maximale pendant 4 minutes

**Procédure de séchage pour obtenir des particules sphériques**

L'émulsion obtenue a été ensuite homogénéisée à une pression de 30 bars au moyen d'une pompe à haute pression puis pulvérisée dans une chambre d'atomisation au moyen d'une buse simultanément avec un courant de $CO_2$

(30 bars, 45°C) que l'on a fait circuler en continu avec un débit d'environ 500g/mn pour éliminer l'eau. La poudre séchée a été retenue sur un filtre situé à la base de la chambre d'atomisation puis collectée après dépressurisation. On obtient ainsi 270g de microcapsules sphériques sous la forme d'une fine poudre blanche ayant un diamètre moyen en nombre de 7,8 $\mu$m et un diamètre moyen en volume de 47 $\mu$m.

La taille des particules a été mesurée en voie sèche par diffraction laser en utilisant un granulomètre Microtrac S3500, les granulométries étant exprimées en volume et en nombre.

| Exemple A | Caractéristiques mesurées des capsules | | | |
|---|---|---|---|---|
| | Taux de parfum encapsulé (%) | Taux de parfum libre (%) | Densité de poudre versée | Densité absolue |
| | 19,8 | < 0,1 | 484 | 1,12 |

**Exemples B à H**

Selon le procédé décrit à l'exemple A, on a préparé les capsules suivantes :

| Composition | Amidon modifié hydrophobe | Polysaccharide hydrosoluble | Parfum de l'exemple A | Eau |
|---|---|---|---|---|
| Exemple B | Amidon Capsul® de National Starch 110g | Maltodextrine MD 120 de Tereos 110g | 55g | 225g |
| Exemple C | Amidon Capsul® de National Starch 110g | Maltodextrine MD 170 de Tereos 110g | 55g | 225g |
| Exemple D | Amidon Capsul® de National Starch | Maltodextrine MD 190 de Tereos | 55g | 225g |

|  |  |  |  |  |
|---|---|---|---|---|
|  | 110g | 110g |  |  |
| **Exemple E** | Amidon Capsul® de National Starch 110g | Maltodextrine de pomme de terre MD 20 P de AVEBE 110g | 105g | 225g |
| **Exemple F** | Amidon Capsul® de National Starch 154g | Maltodextrine de pomme de terre MD 20 P de AVEBE 66g | 55g | 225g |
| **Exemple G** | Amidon Capsul® de National Starch 66g | Maltodextrine de pomme de terre MD 20 P de AVEBE 154g | 55g | 225g |
| **Exemple H (hors invention)** | Amidon Capsul® de National Starch 110g | Sirop de glucose Glucodry G290 de TEREOS 110g | 55g | 225g |

| **Exemples** | **Caractéristiques mesurées des capsules** | | | |
|---|---|---|---|---|
|  | **Taux de parfum encapsulé (%)** | **Taux de parfum libre (%)** | **Densité de poudre versée (g/l)** | **Densité absolue** |
| **Exemple B** | 19,3 | <0,1 | 568 | 1,14 |
| **Exemple C** | 19,4 | <0,1 | 490 | 1,16 |
| **Exemple D** | 19,9 | <0,1 | 537 | 1,11 |
| **Exemple E** | 38 | 0,8 | 482 | 1,08 |
| **Exemple F** | 21,0 | 0,2 | 595 | 1,11 |
| **Exemple G** | 20,7 | 0,2 | 521 | 1,15 |
| **Exemple H (hors invention)** | 19,2 | 0,1 | 568 | 1,12 |

### Exemple I comparatif

On a réalisé des capsules ayant la même composition que l'exemple A tel décrit ci-dessus selon le procédé de l'exemple 1 du brevet US6200949 comprenant un séchage par spray-drying (atomisation) de l'émulsion .

L'émulsion est séchée par spray-drying au moyen d'un appareil du type Bowen Lab Model Dryer utilisant de l'air avec un débit de 420m$^3$/h à une température de 204°C et une température externe de 93°C et une vitesse de la turbine de 50000tr/mn.

Aspect morphologique des particules obtenues: polymorphe avec agrégats.

### Exemple J comparatif

On a réalisé des capsules ayant la même composition que l'exemple A tel décrit ci-dessus selon le procédé de l'exemple 1 du brevet US5508259 comprenant un séchage par spray-drying (atomisation) de l'émulsion.

Le mélange a été séché par spray-drying avec un appareil du type CCM Sulzer avec un débit d'émulsion de 50kg/h, de l'air à un débit de 320m$^3$/h à 350°C et 0,45 bar.

Aspect morphologique des particules obtenues : polymorphe avec agrégats.

| Composition | Caractéristiques mesurées des capsules | | | |
|---|---|---|---|---|
| | Taux de parfum encapsulé (%) | Taux de parfum libre (%) | Densité de poudre versée (g/l) | Densité absolue |
| Exemple I (hors invention) | 18,3 | 2,7 | 259 | 1,16 |
| Exemple J (hors invention) | 11,2 | 1,7 | 269 | 1,12 |

### Exemples de formulations :

### Exemple 1 : Produit déodorant et anti-transpirant

On a préparé un déodorant et antitranspirant de composition suivante :

| Ingrédients (Nom INCI) | Quantités en % en poids |
|---|---|
| ALCOOL BUTYLIQUE OXYPROPYLENE (14 OP) | 5,9 |
| PALMITATE D'ISOPROPYLE | 25,4 |
| DISTEARATE DE POLYETHYLENE GLYCOL (8 OE) | 6,2 |
| COMPLEXE TETRA HYDROXYCHLORURE ALUMINIUM / ZIRCONIUM TAMPONE GLYCINE | 19,0 |
| HYDROXYDE DE CALCIUM | 0,5 |
| CIRE DE POLYETHYLENE (PM: 500) | 11,4 |
| POLY DIMETHYLSILOXANE (VISCOSITE: 10 CST) | 22,2 |
| OZOKERITE | 2,6 |
| PERLITE (25 MICRONS) | 0,2 |
| Capsules de parfum de l'exemple A | 6,7 |
| TOTAL | 100 |

On introduit dans une cuve la palmitate d'isopropyle et l'alcool butylique oxypropylène. On chauffe le mélange à 65°C puis on ajoute les autres ingrédients (un par un) en restant à 65-70°C. On homogénéise l'ensemble jusqu'à obtention d'une solution homogène pendant environ 15 minutes. On ajoute la perlite et les capsules de parfum de l'exemple A puis refroidit à environ 55°C (quelques °C au-dessus de l'épaississement du mélange) et on coule dans des sticks qu'on stocke dans un réfrigérateur à 4°C pendant 30 minutes.

**Exemples C1 et C2:**

Similairement à l'exemple 1, on a préparé des sticks anti-transpirant déodorantes ayant les compositions suivantes :

| Exemple C1 (hors invention) | |
|---|---|
| Ingrédients | (% en poids) |
| ALCOOL BUTYLIQUE OXYPROPYLENE | 5,9 |
| PALMITATE D'ISOPROPYLE | 25,4 |
| DISTEARATE DE POLYETHYLENE GLYCOL | 6,2 |
| COMPLEXE TETRA HYDROXYCHLORURE ALUMINIUM / ZIRCONIUM TAMPONE GLYCINE | 19,0 |
| HYDROXYDE DE CALCIUM | 0,5 |
| CIRE DE POLYETHYLENE | 11,4 |
| PDMS | 22,2 |
| OZOKERITE | 2,6 |
| Perlite | 0,2 |
| Capsules de parfum de l'exemple I | 6,7 |
| TOTAL | 100 |

| Exemple C2 (hors invention) | |
|---|---|
| Ingrédients | (% en poids) |
| ALCOOL BUTYLIQUE OXYPROPYLENE | 5,9 |
| PALMITATE D'ISOPROPYLE | 25,4 |
| DISTEARATE DE POLYETHYLENE GLYCOL | 6,2 |
| COMPLEXE TETRA HYDROXYCHLORURE ALUMINIUM / ZIRCONIUM TAMPONE GLYCINE | 19,0 |
| HYDROXYDE DE CALCIUM | 0,5 |
| CIRE DE POLYETHYLENE | 11,4 |
| PDMS | 22,2 |
| OZOKERITE | 2,6 |
| Perlite | 0,2 |
| Capsules de parfum de l'exemple J | 6,7 |
| TOTAL | 100 |

**Evaluation des exemples**

**Protocole d'évaluation :**

On a déposé de façon homogène environ 0,2 g de composition sur une mouillette (référence chez Granger Veyron : 40140BCSI de taille 4cm x 14cm). Après une minute on a évalué intensité odeur de parfum. Puis on a simulé la transpiration par un ajout d'eau d'environ 0,1g (trois sprays) sur la composition déposée. On a attendu une minute et on a à nouveau senti.

| Formule | Intensité odeur AV | Intensité odeur AP |
|---|---|---|
| Exemple 1 | Inodore | Très forte odeur de parfum |
| Exemple C1 | Forte odeur de parfum | Très forte odeur de parfum |
| Exemple C2 | Forte odeur de parfum | Forte odeur de parfum |
| AV = avant ajout d'eau ; AP = après ajout d'eau ; | | |

On a ainsi observé à $T_0$ que la formule de l'exemple 1 comprenant les capsules de parfum selon l'invention ne présente aucune odeur avant l'ajout d'eau contrairement aux exemples C1 et C2 (hors invention), ce qui montre que les capsules de parfum dans les exemples C1 et C2 ne sont pas étanches avant même l'ajout d'eau.

On a également observé que la composition de l'exemple 1 après stimulation à l'eau, conduisait à une odeur très intense, ce qui montre une libération importante de parfum en réponse au stimuli eau.

**Exemple 2 : Préparation d'un stick anti-transpirant avant la composition suivante** :

| Ingrédients | Quantités en % en poids |
|---|---|
| Cyclopentasiloxane (DC245 -DOW CORNING) | 20,0 |
| Hexyldecyl stearate (Eutanol G16 S - COGNIS) | 14,0 |
| PPG-14 Butyl ether | 5,0 |
| Huile de ricin hydrogénée (Cutina HR-COGNIS) | 6,0 |
| Alcool cétéarylique | 8,0 |
| Cetearyl alcohol/Ceteareth-30 80/20 (Sinnowax AO- COGNIS) | 14,0 |
| Talc | 7,0 |
| MICRODRY ALUMINUM CHLOROHYDRATE® | 20,0 |
| Capsules de parfum de l'exemple G | 6,0 |
| Total | 100 |

Les capsules de parfum de l'exemple G peuvent être remplacées par les capsules des exemples A à F et H décrites précédemment.

On chauffe la cyclopentasiloxane à 65°C. On ajoute les autres ingrédients (1 par 1) en restant à 65-70°C. On homogénéise l'ensemble (solution transparente) pendant 15 minutes. On ajoute les 2 actifs déodorants et les capsules de l'exemple G. On refroidit à environ 55°C (quelques degrés Celcius au-dessus de l'épaississement du mélange) et on coule dans les sticks qu'on met dans un réfrigérateur à 4°C pendant 30 minutes.

On a évalué l'intensité de l'odeur du parfum sur la peau à T0, T2h et T6h après application de la composition. On a appliqué environ 0,15 g de la composition de l'exemple 2 sur la peau. Après une minute on a évalué l'intensité de l'odeur du parfum (AV) en la notant de 0 à 10. Puis on a pulvérisé environ 0,1 g d'eau (trois sprays) sur la composition appliquée sur la peau. On a attendu 30 secondes puis on a évalué l'intensité de l'odeur du parfum (AP) 2 et 6 heures plus tard on a réévalué à nouveau l'intensité de l'odeur (AV) avant un ajout d'eau d'environ 0,1g (trois sprays) sur la composition appliquée sur la peau. On a attendu 30 secondes puis on a évalué l'intensité de l'odeur (AP).

| Produit | Intensité odeur T0h | | | Intensité odeur T2h | | | Intensité odeur T6h | | |
|---|---|---|---|---|---|---|---|---|---|
| | AV | AP | Δ | AV | AP | Δ | AV | AP | Δ |
| Exemple 2 | 1,0 | 5,0 | 4 | 3,0 | 5,5 | 2,5 | 2,5 | 4,5 | 2,0 |
| AV = avant ajout d'eau ; AP = après ajout d'eau ;<br>Δ= amplitude de différence d'intensité olfactive (AV - AP)<br>Echelle d'intensité odeur de parfum : 0 à 10 (0= inodore; 10= odeur très intense / saturée). | | | | | | | | | |

On a ainsi observé à T0 que sur la peau la composition de l'exemple 2 présente une odeur quasi-inodore. On a également observé que chaque pulvérisation d'eau sur le produit à T0, T2h et T6h conduit à une augmentation de l'intensité de l'odeur de parfum, ce qui montre une libération importante de parfum.

**Exemple 3 :**

Similairement à l'exemple 2 on a préparé un stick anti-transpirant ayant la composition suivante :

| Ingrédients | % en poids |
|---|---|
| Cyclopentasiloxane (DC245 -DOW CORNING) | 20,6 |
| Hexyldecyl stearate (Eutanol G16 S - COGNIS) | 14,0 |
| PPG-14 Butyl ether | 5,0 |
| Huile de ricin hydrogénée (Cutina HR-Cognis) | 6,0 |
| Alcool cétéarylique | 8,0 |
| Cetearyl alcohol/Ceteareth-30 80/20 (Sinnowax AO-Cognis) | 14,0 |
| Talc | 7,0 |
| Aluminium Chlorhydrate (Micro Dry) | 20,0 |
| Capsules de parfum de l'exemple A | 5,0 |
| Parfum libre | 0,4 |
| Total | 100 |

Les capsules de parfum de l'exemple A peuvent être remplacées par les capsules des exemples B à H décrites précédemment.

On a évalué l'intensité de l'odeur du parfum sur la peau à T0, T2h et T6h après application de la composition selon le protocole décrit dans l'exemple 2.

| Produit | Intensité odeur T0h | | | Intensité odeur T2h | | | Intensité odeur T6h | | |
|---|---|---|---|---|---|---|---|---|---|
| | AV | AP | Δ | AV | AP | Δ | AV | AP | Δ |
| Exemple 3 | 5,0 | 6,5 | 1,5 | 4,0 | 6,0 | 2,0 | 3,0 | 6,0 | 3,0 |

On a ainsi observé à T0 que la composition de l'exemple 3 présente une odeur de parfum d'intensité moyenne. On a également observé à chaque temps (T0, T2h et T6h) que la pulvérisation d'eau sur le produit conduit à une augmentation de l'intensité de l'odeur de parfum (notamment des notes fraiches), ce qui montre une libération importante de parfum.

**Exemple 4 : Préparation d'un stick anti-transpirant avant la composition suivante** :

| Ingrédients | % en poids |
|---|---|
| CIRE DE POLYETHYLENE (PERFORMALENE 500-L POLYETHYLENE (New Phase technologie) | 4,1 |
| HOMOPOLYMERE DE L'ETHYLENE (PERFORMALENE 400 POLYETHYLENE - New Phase technologie) | 8,3 |
| CYCLOHEXADIMETHYLSILOXANE DOW CORNING 246 FLUID -Dow Corning) | 25,4 |
| PHENYL TRIMETHICONE (DOW CORNING 556 COSMETIC GRADE FLUID-Dow Corning) | 18,6 |
| ISO-HEXADECANE | 19,6 |

(suite)

| Ingrédients | % en poids |
|---|---|
| METHYL METHACRYLATE CROSSPOLYMER GANZPEARL GMP 0820 - Ganz Chemical) | 13,0 |
| EXPANSED MILLED PERLITE (OPTIMAT 1430 OR - Word Minerals) | 4,5 |
| PYRROLIDONE CARBOXYLATE DE ZINC MICRONISE (UCIB -Solabia) | 0,5 |
| Capsules de Parfum de l'exemple G | 6,0 |
| Total | 100 |

Les capsules de parfum de l'exemple G peuvent être remplacées par les capsules des exemples A à F et H décrites précédemment.

On chauffe la cyclohexadimethylsiloxane à 65°C. On ajoute les autres ingrédients (un par un) en restant à 65-70°C. On homogénéise l'ensemble jusqu'à obtention d'une solution transparente pendant environ 15 minutes. On ajoute alors la pyrrolidone carboxylate de zinc, la perlite et les capsules de parfum de l'exemple G puis refroidit à environ 55°C (quelques °C au-dessus de l'épaississement du mélange) et on coule dans des sticks qu'on met à 4°C pendant 30 minutes.

La composition appliquée sur les aisselles laisse un dépôt sur la peau dégageant une odeur de parfum. Le parfum se libère au cours de la journée lorsque les capsules sont au contact de la transpiration.

**Revendications**

1. Composition anhydre solide comprenant:

1) au moins des particules comprenant un cœur contenant au moins un agent bénéfique et une enveloppe entourant le coeur; ladite enveloppe comprenant au moins un polysaccharide modifié hydrophobe choisi parmi les $C_5$-$C_{20}$-alcényl succinate d'amidon et au moins un glucide hydrosoluble choisi parmi les maltodextrines ayant un Dextrose Equivalent allant de 4 à 20 ;
lesdites particules présentant simultanément une densité de poudre versée allant de 300,0 g/l à 600,0 g/l et une densité absolue supérieure à 1.0 ;
les particules étant sphériques et ayant un diamètre moyen en nombre allant de 1 à 30μm et un diamètre moyen en volume allant de 5 à 15μm ; et
2) au moins un agent structurant.

2. Composition selon la revendication 1, comprenant un milieu physiologiquement acceptable.

3. Composition selon la revendication 1 ou 2, où les particules sont sphériques, et en particulier ont un diamètre moyen en nombre allant de 2 à 15 μm et de préférence de 5 à 10 μm et un diamètre moyen en volume allant de 10 à 100 μm et de préférence de 20 à 80 μm.

4. Composition selon l'une quelconque des revendications 1 à 3, où le polysaccharide modifié hydrophobe est l'octényle succinate d'amidon sodique.

5. Composition selon l'une quelconque des revendications 1 à 4, où le polysaccharide modifié hydrophobe représente de 20 à 90% en poids, notamment 30 à 80% en poids, mieux 40 à 70% en poids, et encore mieux 40 à 60% en poids par rapport au poids total de l'enveloppe de la particule.

6. Composition selon l'une quelconque des revendications précédentes, où le glucide hydrosoluble est choisi parmi les maltodextrines ayant un Dextrose Equivalent allant de 12 à 20.

7. Composition selon l'une quelconque des revendications précédentes, où le ou les glucides (s) hydrosoluble(s) représentent de 10 à 80% en poids, de préférence de 15 à 70% en poids, de préférence de 20 à 65% en poids, et encore mieux de 40 à 60% en poids par rapport au poids total de l'enveloppe de la particule.

8. Composition selon l'une des revendications précédentes, où l'enveloppe des particules à libération d'agent bénéfique est constituée

   a) d'au moins un $C_5$-$C_{20}$-alcényl succinate d'amidon dans une quantité allant de 20 à 90% en poids, notamment 30 à 80% en poids, mieux 40 à 70% en poids, et encore mieux 40 à 60% en poids par rapport au poids total de l'enveloppe de la particule et
   b) d'au moins une maltodextrine de D.E. allant de 4 à 20 et de préférence allant de 12 à 20 dans une quantité allant de 10 à 80% en poids, de préférence de 15 à 70% en poids, plus préférentiellement de 20 à 65% en poids, et encore mieux de 40 à 60% en poids par rapport au poids total de l'enveloppe de la particule.

9. Composition selon l'une quelconque des revendications précédentes, où l'agent structurant est choisi parmi la cire d'abeille, la cire de lanoline, la cire de Carnauba, la cire de Candellila, les cires d'orange et de citron, les cires microcristallines, les paraffines, l'ozokérite, les cires de polyéthylène, les triglycérides d'acides gras, l'alcool stéarylique, l'alcool cétéarylique, l'alcool cétylstéarylique, l'huile de jojoba isomérisée, l'huile de tournesol hydrogénée, l'huile de ricin hydrogénée, l'huile de coprah hydrogénée, l'hexyldecyl stearate, un mélange de stérols de soja et de pentaérythritol oxyéthyléné (50E) oxypropyléné (5 OP) et leurs mélanges et encore plus préférentiellement choisi parmi l'alcool cétylstéarylique, les cires de Carnauba, la cire de Candellila, les cires de polyéthylène, l'huile de ricin hydrogénée le mélange de stérols de soja et de pentaérythritol oxyéthyléné (5OE) oxypropyléné (5 OP), l'ozokérite et l'hexyldecyl stearate et leurs mélanges.

10. Composition selon l'une quelconque des revendications précédentes, où les particules à libération d'agent bénéfique sont susceptibles d'être obtenues selon un procédé comprenant au moins les étapes suivantes :

    - on prépare une solution aqueuse constituée du mélange du glucide hydrosoluble du polysaccharide modifié hydrophobe puis on ajoute l'agent bénéfique et on agite de façon à former une émulsion ; et
    - on homogénéise ladite émulsion ainsi formée sous haute pression à une pression allant de 10 à 200 bars et plus préférentiellement de 20 à 200 bars ; et
    - on pulvérise ladite émulsion dans une chambre de séchage, et
    - l'eau est extraite pendant une durée de préférence ne dépassant pas 3 heures, et plus préférentiellement ne dépassant pas 30 minutes, avec un fluide sous pression tel que le dioxyde de carbone, de préférence à l'état supercritique de préférence à une pression d'au moins 0,3XPc et à une température d'au moins Tc-60°C avec Pc correspondant à la pression critique du gaz et Tc la température critique du gaz, de façon à obtenir les particules à libération d'agent bénéfique.

11. Composition selon l'une quelconque des revendications précédentes , où les agents bénéfiques sont choisis parmi :

    (i) les corps gras ;
    (ii) les substances parfumantes ;
    (iii) les principes actifs pharmaceutiques ;
    (iv) les actifs cosmétiques

12. Composition selon l'une quelconque des revendications précédentes, où les agents bénéfiques sont choisis parmi les substances parfumantes et plus particulièrement celles choisies parmi les notes de cœur et/ou de tête et encore plus particulièrement choisies parmi :

    Acétate de benzyle
    Acétate de géranyle
    Acétate de cis-3-hexenyle
    Aldéhyde C18 ou nonalactone
    Acétate de décyle
    Allyl amyl glycolate (citral)
    Acétate d'éthyle
    Acétate de butyle
    Allyl 3-cyclohexylpropionate
    Acétate de linalyle
    Alcool Phényléthylique
    Acétate d'héxyle
    Berryflor ou éthyl-6-(acétyloxy)-hexanoate

Acétate d'isoamyle
Caproate d'allyle
Amarocite ou 6,6-diméthoxy-2,5,5-triméthylhex-2-ène
Citral lemarome N ou 3,7-diméthylocta-2,6-diénal
Canthoxal ou anisyl propanal
Claritone ou 2,4,4,7-tétraméthyloct-6-èn-3-one Ethyl-2méthyl butyrate
Dihydromyrcénol
Cis-3 hexenol
Hédione ou méthyl dihydrojasmonate
L-carvone
Heptanoate d'allyle
Limonène
Néobuténone alpha ou 1-(5,5-diméthyl-1-cyclohexenyl)-pent-4-èn-1-one
Méthylheptènone
Toscanol ou 4-(cyclopropylméthyl)-phénylméthyl éther myrcenol super ou 2-methyl-6-methylideneoct-7-en-2-ol
Decalactone
Acétate de stéaryle
Oxyde de rose
Linalool
Triplal ou 2,4-dimethylcyclohex-3-ène-1-carbaldéhyde
Mélonal ou 2,6-dimethylhept-5-ènal
1-(1,2,3,4,5,6,7,8-octahydro-2,3,8,8-tetraméethyl-2-naphthyl)éthan-1-one
Hexylcinnamal
Tétrahydro-2-isobutyl-4-méthylpyrann-4-ol
Salicylate d'hexyle
1,4-Dioxacycloheptadécane-5,17-dione

et leurs mélanges

13. Composition selon l'une quelconque des revendications précédentes, où les particules comprennent au moins une ou plusieurs substances parfumantes ayant une pression de vapeur saturante à 25°C supérieure ou égale à 10,0 Pa et, de préférence, la ou lesdites susbtances parfumantes représentent de 50 à 100% en poids, plus préférentiellement de 60 à 100% en poids, encore plus préférentiellement de 70 à 100% en poids, et encore mieux de 80 à 100% en poids du poids total des substances parfumantes présentes dans les particules

14. Composition selon l'une quelconque des revendications précédentes, **caractérisée par le fait que**

a) les particules comprennent au moins une substance parfumante et
b) la composition comprend en plus au moins une substance parfumante sous forme libre, identique ou différente de ladite substance parfumante présente dans lesdites particules.

15. Composition selon l'une quelconque des revendications précédentes, **caractérisée par le fait qu'**elle contient exclusivement une ou plusieurs substances parfumantes encapsulées dans les particules.

16. Composition selon l'une quelconque des revendications précédentes, comprenant au moins un agent de coloration notamment choisi parmi les nacres, les pigments, les particules réfléchissantes, et leur(s) mélange(s) et où, plus particulièrement, les particules à libération d'agent bénéfique comprennent au moins une substance parfumante, et encore plus particulièrement, où la composition comprend en plus au moins une substance parfumante sous forme libre, identique ou différente de ladite substance parfumante présente dans lesdites particules.

17. Composition selon l'une quelconque des revendications précédentes, comprenant au moins un actif déodorant et/ou au moins un actif anti-transpirant sous forme libre et/ou sous forme encapsulée et où, plus particulièrement, les particules à libération d'agent bénéfique comprennent au moins une substance parfumante, et encore plus particulièrement, où la composition comprend en plus au moins une substance parfumante sous forme libre, identique ou différente de ladite substance parfumante présente dans lesdites particules.

18. Procédé de soin et/ou d'hygiène et/ou de de conditionnement et/ou de parfumage et/ou de maquillage d'une matière kératinique humaine consistant à appliquer sur ladite matière kératinique humaine une composition selon l'une quelconque des revendications précédentes.

19. Procédé cosmétique de traitement des odeurs corporelles et éventuellement de la transpiration humaine consistant à appliquer sur une matière kératinique une composition selon la revendication 17.

20. Produit de consommation, **caractérisé par le fait qu'**il est constitué par une composition selon l'une quelconque des revendications 1 à 17.

**Patentansprüche**

1. Feste wasserfreie Zusammensetzung, umfassend

1) mindestens Teilchen mit einem Kern, der mindestens einen Effektstoff enthält, und einer den Kern umgebenden Hülle; wobei die Hülle mindestens ein hydrophob modifiziertes Polysaccharid, das aus Stärke-$C_5$-$C_{20}$-alkenylsuccinat ausgewählt ist, und mindestens ein wasserlösliches Kohlenhydrat, das aus Maltodextrinen mit einem Dextroseäquivalent im Bereich von 4 bis 20 ausgewählt ist, umfasst; wobei die Teilchen gleichzeitig eine Pulverschüttdichte im Bereich von 300,0 g/l bis 600,0 g/l und eine absolute Dichte von mehr als 1,0 aufweisen; wobei die Teilchen kugelförmig sind und einen zahlenmittleren Durchmesser im Bereich von 1 bis 30 $\mu$m und einen volumenmittleren Durchmesser im Bereich von 5 bis 150 $\mu$m aufweisen; und
2) mindestens ein Strukturierungsmittel.

2. Zusammensetzung nach Anspruch 1, umfassend ein physiologisch unbedenkliches Medium.

3. Zusammensetzung nach Anspruch 1 oder 2, wobei die Teilchen kugelförmig sind und insbesondere einen zahlenmittleren Durchmesser im Bereich von 2 bis 15 $\mu$m und vorzugsweise von 5 bis 10 $\mu$m und einen volumenmittleren Durchmesser im Bereich von 10 bis 100 $\mu$m und vorzugsweise von 20 bis 80 $\mu$m aufweisen.

4. Zusammensetzung nach einem der Ansprüche 1 bis 3, wobei es sich bei dem hydrophob modifizierten Polysaccharid um Natriumstärkeoctenylsuccinat handelt.

5. Zusammensetzung nach einem der Ansprüche 1 bis 4, wobei das hydrophob modifizierte Polysaccharid 20 bis 90 Gew.-%, insbesondere 30 bis 80 Gew.-%, besser 40 bis 70 Gew.-% und noch besser 40 bis 60 Gew.-%, bezogen auf das Gesamtgewicht der Hülle des Teilchens, ausmacht.

6. Zusammensetzung nach einem der vorhergehenden Ansprüche, wobei das wasserlösliche Kohlenhydrat aus Maltodextrinen mit einem Dextroseäquivalent im Bereich von 12 bis 20 ausgewählt ist.

7. Zusammensetzung nach einem der vorhergehenden Ansprüche, wobei das wasserlösliche Kohlenhydrat bzw. die wasserlöslichen Kohlenhydrate 10 bis 80 Gew.-%, vorzugsweise 15 bis 70 Gew.-%, vorzugsweise 20 bis 65 Gew.-% und noch besser 40 bis 60 Gew.-%, bezogen auf das Gesamtgewicht der Hülle des Teilchens, ausmacht bzw. ausmachen.

8. Zusammensetzung nach einem der vorhergehenden Ansprüche, wobei die Hülle der Teilchen mit Freisetzung von Effektstoff aus

a) mindestens einem Stärke-$C_5$-$C_{20}$-alkenylsuccinat in einer Menge im Bereich von 20 bis 90 Gew.-%, insbesondere 30 bis 80 Gew.-%, besser 40 bis 70 Gew.-% und noch besser 40 bis 60 Gew.-%, bezogen auf das Gesamtgewicht der Hülle des Teilchens, und
b) mindestens einem Maltodextrin mit einem D.E. im Bereich von 4 bis 20 und vorzugsweise im Bereich von 12 bis 20 in einer Menge im Bereich von 10 bis 80 Gew.-%, vorzugsweise von 15 bis 70 Gew.-%, weiter bevorzugt von 20 bis 65 Gew.-% und noch besser von 40 bis 60 Gew.-%, bezogen auf das Gesamtgewicht der Hülle des Teilchens,

besteht.

9. Zusammensetzung nach einem der vorhergehenden Ansprüche, wobei das Strukturierungsmittel aus Bienenwachs, Lanolinwachs, Carnaubawachs, Candelillawachs, Orangen- und Zitronenwachs, mikrokristallinen Wachsen, Paraffinen, Ozokerit, Polyethylenwachsen, Fettsäuretriglyceriden, Stearylalkohol, Cetearylalkohol, Cetylstearylalkohol, isomerisiertem Jojobaöl, hydriertem Sonnenblumenöl, hydriertem Rizinusöl, hydriertem Kokosnussöl, Hexyldecylstearat, einer Mischung von Sojasterolen und oxyethyleniertem (5 EO) oxypropyleniertem (5 PO) Pentaerythrit und Mischungen davon und noch weiter bevorzugt aus Cetylstearylalkohol, Carnaubawachsen, Candelillawachs, Polyethylenwachsen, hydriertem Rizinusöl, der Mischung von Sojasterolen und oxyethyleniertem (5 EO) oxypropyleniertem (5 PO) Pentaerythrit, Ozokerit und Hexyldecylstearat und Mischungen davon ausgewählt ist.

10. Zusammensetzung nach einem der vorhergehenden Ansprüche, wobei die Teilchen mit Freisetzung von Effektstoff gemäß einem Verfahren erhältlich sind, das mindestens die folgenden Schritte umfasst:

- man stellt eine wässrige Lösung her, die aus der Mischung des wasserlöslichen Kohlenhydrats des hydrophob modifizierten Polysaccharids besteht, gibt dann den Effektstoff zu und rührt zur Bildung einer Emulsion; und
- man homogenisiert die so gebildete Emulsion unter hohem Druck bei einem Druck im Bereich von 10 bis 200 bar und weiter bevorzugt von 20 bis 200 bar; und
- man versprüht die Emulsion in einer Trocknungskammer; und
- man extrahiert das Wasser über einen Zeitraum, der vorzugsweise nicht mehr als 3 Stunden und weiter bevorzugt nicht mehr als 30 Minuten beträgt, mit einem unter Druck stehenden Fluid wie Kohlendioxid, vorzugsweise in überkritischem Zustand, vorzugsweise bei einem Druck von mindestens 0,3xPc und einer Temperatur von mindestens Tc-60 °C, wobei Pc dem kritischen Druck des Gases entspricht und Tc dem kritischen Druck des Gases entspricht, wodurch man die Teilchen mit Freisetzung von Effektstoff erhält.

11. Zusammensetzung nach einem der vorhergehenden Ansprüche, wobei die Effektstoffe aus

(i) Fettsubstanzen;
(ii) Parfümierungssubstanzen;
(iii) pharmazeutischen Wirkstoffen;
(iv) kosmetischen Wirkstoffen

ausgewählt sind.

12. Zusammensetzung nach einem der vorhergehenden Ansprüche, wobei die Effektstoffe aus Parfümierungssubstanzen ausgewählt sind und spezieller aus jenen, die aus Herz- und/oder Kopfnoten und noch spezieller aus

Benzylactetat
Geranylacetat
cis-3-Hexenylacetat
$C_{18}$-Aldehyd oder Nonalacton
Decylacetat
Allylamylglykolat (Citral)
Ethylacetat
Butylactetat
Allyl-3-cyclohexylpropionat
Linalylacetat
Phenylethylalkohol
Hexylacetat
Berryflor oder Ethyl-6-(acetyloxy)hexanoat
Isoamylacetat
Allylcaproat
Amarocite oder 6,6-Dimethoxy-2,5,5-trimethylhex-2-en
Citral lemarome N oder 3,7-Dimethylocta-2,6-dienal
Canthoxal oder Anisylpropanal
Claritone oder 2,4,4,7-Tetramethyloct-6-en-3-on Ethyl-2-methylbutyrat
Dihydromyrcenol
cis-3-Hexenol
Hedion oder Methyldihydrojasmonat
L-Carvon
Allylheptanoat

Limonen
Neobutenon alpha oder 1-(5,5-Dimethyl-1-cyclohexenyl)pent-4-en-1-on
Methylheptenon
Toscanol oder 4-(Cyclopropylmethyl)phenylmethylether
Myrcenol super oder 2-Methyl-6-methylidenoct-7-en-2-ol
Decalacton
Stearylacetat
Rosenoxid
Linalool
Triplal oder 2,4-Dimethylcyclohex-3-en-1-carbaldehyd
Melonal oder 2,6-Dimethylhept-5-enal 1-(1,2,3,4,5,6,7,8-Octahydro-2,3,8,8-tetramethyl-2-naphthyl)ethan-1-on
Hexylcinnamal
Tetrahydro-2-isobutyl-4-methylpyran-4-ol
Hexylsalicylat
1,4-Dioxacycloheptadecan-5,17-dion
und Mischungen davon
ausgewählt sind.

13. Zusammensetzung nach einem der vorhergehenden Ansprüche, wobei die Teilchen mindestens einen oder mehrere Parfümierungssubstanzen mit einem Sättigungsdampfdruck bei 25 °C größer oder gleich 10,0 Pa aufweisen und die Parfümierungssubstanz bzw. die Parfümierungssubstanzen vorzugsweise 50 bis 100 Gew.-%, weiter bevorzugt 60 bis 100 Gew.-%, noch weiter bevorzugt 70 bis 100 Gew.-% und noch besser 80 bis 100 Gew.-% des Gesamtgewichts der in den Teilchen vorliegenden Parfümierungssubstanzen ausmacht bzw. ausmachen.

14. Zusammensetzung nach einem der vorhergehenden Ansprüche, **dadurch gekennzeichnet, dass**

a) die Teilchen mindestens eine Parfümierungssubstanz umfassen und
b) die Zusammensetzung außerdem mindestens eine Parfümierungssubstanz in freier Form, die mit der in den Teilchen vorliegenden Parfümierungssubstanz identisch oder davon verschieden ist, umfasst.

15. Zusammensetzung nach einem der vorhergehenden Ansprüche, **dadurch gekennzeichnet, dass** sie ausschließlich eine oder mehrere in den Teilchen verkapselte Parfümierungssubstanzen enthält.

16. Zusammensetzung nach einem der vorhergehenden Ansprüche, umfassend mindestens ein Farbmittel, das insbesondere aus Perlglanzmitteln, Pigmenten, reflektierenden Partikeln und einer Mischung bzw. Mischungen davon ausgewählt ist, und spezieller wobei die Teilchen mit Freisetzung von Effektstoff mindestens eine Parfümierungssubstanz umfassen und noch spezieller wobei die Zusammensetzung außerdem mindestens eine Parfümierungssubstanz in freier Form, die mit der in den Teilchen vorliegenden Parfümierungssubstanz identisch oder davon verschieden ist, umfasst.

17. Zusammensetzung nach einem der vorhergehenden Ansprüche, umfassend mindestens einen Deodorantwirkstoff und/oder mindestens einen Antitranspirantwirkstoff in freier Form und/oder in verkapselter Form, und spezieller wobei die Teilchen mit Freisetzung von Effektstoff mindestens eine Parfümierungssubstanz umfassen und noch spezieller wobei die Zusammensetzung außerdem mindestens eine Parfümierungssubstanz in freier Form, die mit der in den Teilchen vorliegenden Parfümierungssubstanz identisch oder davon verschieden ist, umfasst.

18. Verfahren zur Pflege und/oder zur Hygiene und/oder zur Konditionierung und/oder zur Parfümierung und/oder zum Schminken eines menschlichen Keratinmaterials, das darin besteht, dass man auf das menschliche Keratinmaterial eine Zusammensetzung nach einem der vorhergehenden Ansprüche aufbringt.

19. Kosmetisches Verfahren zur Behandlung von Körpergerüchen und gegebenenfalls menschlicher Transpiration, das darin besteht, dass man eine Zusammensetzung nach Anspruch 17 auf ein Keratinmaterial aufbringt.

20. Konsumprodukt, **dadurch gekennzeichnet, dass** es aus einer Zusammensetzung nach einem der Ansprüche 1 bis 17 besteht.

**Claims**

1. Solid anhydrous composition comprising:

   1) at least particles comprising a core containing at least one beneficial agent and a shell surrounding the core; the said shell comprising at least one hydrophobic-modified polysaccharide chosen from starch $C_5$-$C_{20}$-alkenyl succinate and at least one water-soluble carbohydrate chosen from maltodextrins having a Dextrose Equivalent ranging from 4 to 20; the said particles simultaneously exhibiting a poured powder density ranging from 300.0 g/l to 600.0 g/l and an absolute density of greater than 1.0; the particles being spherical and having a number-average diameter ranging from 1 to 30 $\mu$m and a volume-average diameter ranging from 5 to 150 $\mu$m; and
   2) at least one structuring agent.

2. Composition according to Claim 1, comprising a physiologically acceptable medium.

3. Composition according to Claim 1 or 2, where the particles are spherical and in particular have a number-average diameter ranging from 2 to 15 $\mu$m and preferably from 5 to 10 $\mu$m and a volume-average diameter ranging from 10 to 100 $\mu$m and preferably from 20 to 80 $\mu$m.

4. Composition according to any one of Claims 1 to 3, where the hydrophobic-modified polysaccharide is starch sodium octenyl succinate.

5. Composition according to any one of Claims 1 to 4, where the hydrophobic-modified polysaccharide represents from 20% to 90% by weight, in particular from 30% to 80% by weight, better still from 40% to 70% by weight and even better still from 40% to 60% by weight, with respect to the total weight of the shell of the particle.

6. Composition according to any one of the preceding claims, where the water-soluble carbohydrate is chosen from maltodextrins having a Dextrose Equivalent ranging from 12 to 20.

7. Composition according to any one of the preceding claims, where the water-soluble carbohydrate(s) represent from 10% to 80% by weight, preferably from 15% to 70% by weight, preferably from 20% to 65% by weight and better still from 40% to 60% by weight, with respect to the total weight of the shell of the particle.

8. Composition according to one of the preceding claims, where the shell of the particles having release of beneficial agent is constituted:

   a) of at least one starch $C_5$-$C_{20}$-alkenyl succinate in an amount ranging from 20% to 90% by weight, in particular from 30% to 80% by weight, better still from 40% to 70% by weight and even better still from 40% to 60% by weight, with respect to the total weight of the shell of the particle, and
   b) of at least one maltodextrin with a DE ranging from 4 to 20 and preferably ranging from 12 to 20 in an amount ranging from 10% to 80% by weight, preferably from 15% to 70% by weight, more preferentially from 20% to 65% by weight and better still from 40% to 60% by weight, with respect to the total weight of the shell of the particle.

9. Composition according to any one of the preceding claims, where the structuring agent is chosen from beeswax, lanolin wax, carnauba wax, candelilla wax, orange and lemon waxes, microcrystalline waxes, paraffins, ozokerite, polyethylene waxes, fatty acid triglycerides, stearyl alcohol, cetearyl alcohol, cetylstearyl alcohol, isomerized jojoba oil, hydrogenated sunflower oil, hydrogenated castor oil, hydrogenated coconut oil, hexyldecyl stearate, a mixture of soybean sterols and of oxyethylenated (5 OE)/oxypropylenated (5 OP) pentaerythritol, and their mixtures, and more preferentially still chosen from cetylstearyl alcohol, carnauba waxes, candelilla wax, polyethylene waxes, hydrogenated castor oil, the mixture of soybean sterols and of oxyethylenated (5 OE)/oxypropylenated (5 OP) pentaerythritol, ozokerite and hexyldecyl stearate, and their mixtures.

10. Composition according to any one of the preceding claims, where the particles having release of beneficial agent are capable of being obtained according to a process comprising at least the following stages:

    - an aqueous solution constituted of the mixture of water-soluble carbohydrate of the hydrophobic-modified polysaccharide is prepared, the beneficial agent is then added and stirring is carried out so as to form an emulsion; and

- the said emulsion thus formed is homogenized under high pressure at a pressure ranging from 10 to 200 bar and more preferentially from 20 to 200 bar; and

- the said emulsion is sprayed into a drying chamber; and

- the water is extracted over a period of time preferably not exceeding 3 hours and more preferentially not exceeding 30 minutes with a pressurized fluid, such as carbon dioxide, preferably in the supercritical state, preferably at a pressure of at least $0.3 \times$ Pc and at a temperature of at least Tc-60°C, with Pc corresponding to the critical pressure of the gas and Tc corresponding to the critical temperature of the gas, so as to obtain the particles having release of beneficial agent.

11. Composition according to any one of the preceding claims, where the beneficial agents are chosen from:

(i) fatty substances;
(ii) fragrancing substances;
(iii) pharmaceutical active principles;
(iv) cosmetic active principles.

12. Composition according to any one of the preceding claims, where the beneficial agents are chosen from fragrancing substances and more particular those chosen from the heart and/or top notes and more particularly still chosen from:

Benzyl acetate
Geranyl acetate
cis-3-Hexenyl acetate
$C_{18}$ Aldehyde or nonalactone
Decyl acetate
Allyl amyl glycolate (citral)
Ethyl acetate
Butyl acetate
Allyl 3-cyclohexylpropionate
Linalyl acetate
Phenylethyl alcohol
Hexyl acetate
Berryflor or ethyl 6-(acetyloxy)hexanoate
Isoamyl acetate
Allyl caproate
Amarocite or 6,6-dimethoxy-2,5,5-trimethylhex-2-ene
Citral lemarome N or 3,7-dimethylocta-2,6-dienal
Canthoxal or anisyl propanal
Claritone or 2,4,4,7-tetramethyloct-6-en-3-one.
Ethyl 2-methylbutyrate
Dihydromyrcenol
cis-3-Hexenol
Hedione or methyl dihydrojasmonate
L-Carvone
Allyl heptanoate
Limonene
Neobutenone alpha or 1-(5,5-dimethyl-1-cyclohexenyl)pent-4-en-1-one
Methylheptenone
Toscanol or 4-(cyclopropylmethyl)phenyl methyl ether
Myrcenol Super or 2-methyl-6-methylideneoct-7-en-2-ol
Decalactone
Stearyl acetate
Rose oxide
Linalool
Triplal or 2,4-dimethylcyclohex-3-ene-1-carbaldehyde
Melonal or 2,6-dimethylhept-5-enal
1-(1,2,3,4,5,6,7,8-Octahydro-2,3,8,8-tetramethyl-2-naphthyl)ethan-1-one
Hexylcirmamal

Tetrahydro-2-isobutyl-4-methylpyran-4-ol
Hexyl salicylate
1,4-Dioxacycloheptadecane-5,17-dione
and their mixtures.

13. Composition according to any one of the preceding claims, where the particles comprise at least one or more fragrancing substances having a saturated vapour pressure at 25°C of greater than or greater than 10.0 Pa and, preferably, the said fragrancing substance(s) represent from 50% to 100% by weight, more preferentially from 60% to 100% by weight, more preferentially still from 70% to 100% by weight and better still from 80% to 100% by weight of the total weight of the fragrancing substances present in the particles.

14. Composition according to any one of the preceding claims, **characterized in that**:

a) the particles comprise at least one fragrancing substance and
b) the composition additionally comprises at least one fragrancing substance in the free form, identical to or different from the said fragrancing substance present in the said particles.

15. Composition according to any one of the preceding claims, **characterized in that** it exclusively contains one or more fragrancing substances encapsulated in the particles.

16. Composition according to any one of the preceding claims, comprising at least one colouring agent chosen in particular from pearlescent agents, pigments and reflective particles, and their mixture(s), and where more particularly the particles having release of beneficial agent comprise at least one fragrancing substance and more particularly still where the composition additionally comprises at least one fragrancing substance in the free form, identical to or different from the said fragrancing substance present in the said particles.

17. Composition according to any one of the preceding claims, comprising at least one deodorant active principle and/or at least one antiperspirant active principle in the free form and/or in the encapsulated form and where more particularly the particles having release of beneficial agent comprise at least one fragrancing substance and more particularly still where the composition additionally comprises at least one fragrancing substance in the free form, identical to or different from the said fragrancing substance present in the said particles.

18. Method for caring for and/or for the hygiene of and/or for conditioning and/or for fragrancing and/or for making up a human keratinous substance, which consists in applying, to the said human keratinous substance, a composition according to any one of the preceding claims.

19. Cosmetic method for the treatment of body odours and optionally of perspiration of human beings, which consists in applying, to a keratinous substance, a composition according to Claim 17.

20. Consumer product, **characterized in that** it is constituted of a composition according to any one of Claims 1 to 17.

**EP 3 166 580 B1**

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 2008156236 A
- US 5508259 A
- US 6200949 B
- EP 1917098 B1
- FR 2792190 A
- WO 02056847 A
- WO 0247619 A
- US 5783657 A
- US 5874069 A
- US 5919441 A
- US 6051216 A
- US 5981680 A

- JP 09188830 A
- JP 10158450 A
- JP 10158541 A
- JP 07258460 A
- JP 05017710 A
- US 5002698 A
- US 2005063928 A
- US 2005084464 A
- US 2005084474 A
- EP 1658863 A
- US 6916465 B

**Littérature non-brevet citée dans la description**

- *CHEMICAL ABSTRACTS,* 66829-29-6
- Silica silylate. CTFA, 2000
- Silica diméthyl silylate. CTFA, 2000
- **S. ARCTANDER.** *Perfume and Flavor Chemicals,* 1969
- **S. ARCTANDER.** *Perfume and Flavor Materials of Natural Origin,* 1960

- Flavor and Fragrance Materials. Allured Publishing Co, 1991
- International Cosmetic Ingredient Dictionnary and Handbook. The Cosmetic, Toiletry, and Fragrance Association, 1997, 371-386, 524-528
- *CHEMICAL ABSTRACTS,* 51981-21-6